(19) Europäisches Patentamt
European Patent Office
Office européen des brevets

(11) EP 0 708 651 B1

## (12) EUROPEAN PATENT SPECIFICATION

(45) Date of publication and mention
of the grant of the patent:
**20.10.1999 Bulletin 1999/42**

(21) Application number: **94918690.2**

(22) Date of filing: **24.06.1994**

(51) Int. Cl.$^6$: **A61K 35/78**, A61K 31/365,
A61K 31/34, A61K 31/19,
A61K 31/12, A61K 31/16,
A61K 31/40

(86) International application number:
**PCT/AU94/00342**

(87) International publication number:
**WO 95/00157** (05.01.1995 Gazette 1995/02)

(54) **THERAPEUTIC AGENT**

THERAPEUTISCHES AGENS

AGENT THERAPEUTIQUE

(84) Designated Contracting States:
**AT BE CH DE DK ES FR GB GR IT LI NL SE**

(30) Priority: **25.06.1993 AU PL960593**

(43) Date of publication of application:
**01.05.1996 Bulletin 1996/18**

(73) Proprietor:
**Main Camp Marketing Pty. Ltd.
Ballina, New South Wales 2478 (AU)**

(72) Inventor: **DAUNTER, Brian
Bellbowrie, QLD 4070 (AU)**

(74) Representative:
**Schwarz, Albin, Dr.
Kopecky & Schwarz
Patentanwälte
Wipplingerstrasse 32/22
1010 Wien (AT)**

(56) References cited:
EP-A- 0 147 778          EP-A- 0 365 210
EP-A- 0 488 965          EP-A- 0 516 588
WO-A-87/01698            WO-A-91/06863
FR-A- 2 334 368          FR-A- 2 611 502
US-A- 4 524 067          US-A- 4 868 153

- **PATENT ABSTRACTS OF JAPAN vol. 011, no.
  106 (C-414) & JP 61 257155 A (HORIUCHI), 14
  November 1986,**
- **C. MACKU ET AL.: "Application of simultaneous
  purging and solvent extraction technique for
  flavour monitoring of natural products." FOOD
  CHEM., vol. 42, no. 2, 1991, pages 121-127,
  XP002050240**
- **DERWENT ABSTRACT; Accession No. 86-
  187794, class B04; & JP,A,61 122 221 (OSAKA
  YAKUHIN KEN K), 10 June 1986.**
- **JOURNAL OF AGRICULTURAL AND FOOD
  CHEMISTRY, Volume 40, No. 6, issued 1992,
  (NITZ, S. et al.), "3-butyl-5,6-dihydro-4H-
  isobenzofuran-1-one , a sensorial active
  phthalide in parsley roots", pages 1038-1040.**
- **THE CHEMISTRY OF ESSENTIAL OILS AND
  ARTIFICIAL PERFUMES, (PARRY, E.J.), 2nd
  Edition, published by SCOTT GREENWOOD &
  SON. 1908, pages 345-347 and 350-353.**
- **JOURNAL OF ASSOCIATION OF OFFICIAL
  ANALYTICAL CHEMISTS INC., Volume 61, No. 5,
  issued 1978, (Lund, E.D.): "Thin layer and high
  pressure liquid chromatographic analysis of
  celery seed oil", pages 1083-1088.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- JOURNAL OF ORGANIC CHEMISTRY, Volume 42, No. 13, issued 1977, (BJELDANES, L.F. et al.), "Phthalide components of celery essential oil", pages 2333-2335.
- JOURNAL OF FOOD SCIENCE, Volume 43, No. 1, issued 1978, (BJELDANES, L.F. et al.), "Sedative activity of celery oil constituents", page 143.

- DERWENT ABSTRACT; Accession No. 83-818134, Class B04; & JP,A,58 172 321 (DAINIPPON PHARM KK), 11 October 1983.

**Description**

FIELD OF THE INVENTION

THIS INVENTION relates to a therapeutic agent which may be used for the treatment of inflammatory complaints such as arthritis.

BACKGROUND OF THE INVENTION

Arthritis means joint inflammation and really refers to more than 100 rheumatic diseases in which one or more joints are inflamed. Rheumatic illnesses include not only arthritis, but illnesses in which there are aches and pains of the musculoskeletal system, joints, muscles, ligaments, tendons and bursae. Rheumatic syndromes can be classified as inflammatory or degenerative, but this is sometimes less distinct than the nomenclature implies. That is, a joint damaged by inflammatory disease is more vulnerable to degenerative factors, thus syndromes that appear to be degenerative may be associated with inflammation. Hence rheumatic syndromes can virtually present manifestations of numerous types of illnesses. In addition, rheumatic illnesses can be associated with other diseases. This complexity therefore makes diagnosis difficult and its classification tentative (discussed in reference (1) of list of references attached hereto).

The difficulty in diagnosis is demonstrable by the latest consideration given to this complex problem by Webb and Nash in 1990 (2) and similarly this applies to the complexity of classification based on the American Rheumatism Association. It therefore follows that the treatment of rheumatic disease is also complex. This complexity has been addressed by Kremer (3) in 1990, who considers the traditional treatment for rheumatoid arthritis, and proposes modifications to it, and the use of combined chemotherapy and also an experimental approach. These complexities in treatment also apply to other rheumatic diseases such as osteoarthritis (1).

Arthritis can be associated with infectious agents such as bacteria, fungi and viruses. Thus there is gonococcal arthritis, tuberculous arthritis, syphilitic arthritis, mycotic arthritis and viral arthritis. There is also polyarthritis and viral arthritis. There is also polyarthritis of unknown etiology in which there is prominent involvement of spinal articulations, sacroiliac joints and paravertebral soft tissues. There is neuropathic joint disease which is a progressive degenerative arthropathy classified under miscellaneous forms of arthritis which also includes chondrocalcinosis. Chondrocalcinosis is defined as the presence of calcium containing salts in cartilaginous structures of one or more joints. Because calcium salt crystals are found in joint fluid with attendant chronic synovitis, it has been referred to as pseudogout (1).

Rheumatoid arthritis is a systemic disease of unknown cause, although it does lead to autoimmune expression. The clinical and pathologic findings and resultant disability are the result of chronic inflammation of synovial membranes. The inflamed and hyperplastic synovium contributes to cartilage destruction. It appears that the resident macrophage and fibroblast type cells in the synovium become activated and release mediators of an inflammatory response. In addition, rheumatoid arthritis appears to be exacerbated by an immunological stimulated influx of monocytes/macrophages and leucocytes into the synovial fluid.

There are also variants of the rheumatoid arthritis syndrome (1). In addition, there is the problem of defining or judging disease activity, and this applies to rheumatoid arthritis, the complexity of developing and applying a disease activity score is exemplified in the latest article by Van der Heijde et al, 1990 (4).

Osteoarthritis, a degenerative joint disease of articular cartilage, probably begins in the second decade of life. It may be considered as a response to aging but can be accelerated by unknown causes. Whereas rheumatoid arthritis is initiated by an inflammatory response which can lead to the destruction of articular cartilage, osteoarthritis is initiated by the destruction of or lack of repair of articular cartilage. This can lead to an inflammatory joint response, but rarely do the pathologic features of inflammation mimic those of rheumatoid arthritis. In addition, subsequent to ulceration of cartilage, new bone formation occurs at the margin of articular cartilage. These marginal osteophytes are the characteristic boney "spars". Osseous changes also include cysts Of varying sizes beneath the joint and remodelling of subchondral bone (1).

As with rheumatoid arthritis there are also variants of the osteoarthritis syndrome such as calcific tendonitis and bursitis which results in the most common cause from calcium deposits in the rotor tendons and inflammation of the bursae which are closed synovial sacs located at sites of friction between skin, ligaments, tendons, muscles and bones. These constitute the group of non-articular rheumatism (1).

Gout represents a group of genetic diseases and acquired disorders of purine metabolism or excretion that may be identified by hyperuricemia (increase in blood uric acid). Therefore gout is an heterogenous collection of disorders, and different disease states leads to the common feature of persistent hyperuricemia. Also, hyperuricemia may occur following chemotherapy for malignancy disorders due to the destruction of tissue resulting in uric acid overload due to an increase in purines (1).

Uric acid is not toxic in its soluble form, but causes problems when precipitated as sodium urate at pH 7.4 or as uric acid in acidic urine. The precipitation of uric acid as sodium urate may occur in any tissue and results in tophi with tissue disruption and destruction. Its precipitation in the kidney can result in varying degrees of renal impairment. Its precipitation as microcrystals in synovial tissues and spaces leads to acute inflammatory arthritis also known as gouty arthritis (1).

Hyperuricemia and gouty arthritis have also been observed following heart transplantation (5).

Gout is therefore an inflammatory arthritis induced by urate crystals (monosodium urate crystals). The mechanisms by which these monosodium urate crystals (MSUC) lead to synovitis and joint destruction have not been completely elucidated. Nevertheless, eicosanoids have been implicated which are important mediators of inflammatory responses, and are produced in response to MSUC (6). Elevated concentrations of eicosanoids are found in gouty synovial fluid (7, 8), prostaglandin (PGE2) which stimulates bone reabsorption (9) and hydroxyeicosatetrenoic acids are produced by synovial cells exposed to MSUC (10-14). Also, leukotriene B4 is present which stimulates neutrophil infiltration (15). It appears that the rate limiting step in eicosanoid - production is the production of phospholipase enzymes which cleave fatty acids from membrane phospholipids (16). These enzymes are produced by monocytes and neutrophils stimulated by MSUC via a phospholipase activating protein which can be inhibited by colchicine and indomethacin (16). Whether or not asymptomatic gout, in terms of the immunological mechanisms which precede rheumatoid arthritis and osteoarthritis has not been elucidated. In this respect it is interesting to note that hyperuricemia is not a disease but a risk factor for gout and coronary heart disease (17). In addition, MSUC may be present in synovial fluid in the absence of hyperuricemia (1, 18, 19), therefore making the diagnosis of gout difficult.

Various treatments have been proposed for treatment of inflammatory complaints but they have only met with partial success and in this regard have not proved to be satisfactory as described in Sany (23). For example, in relation to rheumatoid arthritis various therapies have been proposed which include either slow acting drugs which act mainly but not exclusively on macrophages (i.e. gold salts) or on T CD4+ cells (i.e. D penicillamine) or immunostimulating agents (i.e. Lemamisole) or immunosuppressive drugs such as alkylating agents and antimetabolites. Aspirin as an analgesic and NSAIDS (non steroidal anti-inflammatory drugs) have also been proposed as well as methotrexate as described in Kremer (3). All these therapies have associated side effects of various severity.

Reference may also be made to references (19), (16), (24), (18), (25), (26), (17), (27), (4) and (5) which also discuss this subject.

The treatment of gout may also be problematic. The use of the anti-inflammatory agents, phenylbutazone or indomethacin can result in headaches and gastrointestinal symptoms (nausea, vomiting, aodominal pain and diarrhoea), but less than that experienced with colchicine which may also induce leucopoenia. Glucocorticoid or ACTH give variable responses and there is a high incidence of rebound effects (1).

Reference may be made to prior art which includes Japanese patent JP1157915 which described a superoxide dismutase (SOD) inducer containing celery or lovage extracts which may be extracted by distillation with steam or with organic solvents by a batch or reflux method. The extract is used at any concentration or also as a dried substance. A surfactant or lactose may be added.

French patent No. 2611502 refers to an extract of roots from the plant family *Umbelliferae* which has diuretic, cardiovascular, analgesic and anti-inflammatory activity. The roots are extracted with an alcoholic solvent and dried to a powder or used as a preservative.

German patent No. 3638484 refers to a plant extract from parsley in an aqueous or alcoholic medium for use as a medicinal anti-lice shampoo.

Japanese patent JP1257155 refers to anti-oxidant food containing a powdered extract of a plant belonging to the *Umbelliferae* family.

Japanese patents JP57056416 and 587058327 refer to mouthwash compositions containing an alcoholic extract of celery.

Russian patent SU639552 refers to an alcoholic extract of parsley juice as an obstetric composition.

Reference may also be made to Japanese patent specifications 63-90505 and 63-90506 which relate to a novel polysaccharide derived from rhizome of *Bupleurum falcatum* belonging to the *Umbelliferae* plant family and effective as a therapeutic agent in relation to autoimmune diseases such as arthrorheumatism, thyroiditis and chronic nephritis.

Extracts from the seeds of the celery plant *Apium graveolens* (family *Umbelliferae*) have been asserted to have pharmacological properties. These range from carminative, diuretic, emmenagogic (an agent that facilitates menstrual discharge) and antiseptic effects. In addition such extracts have also been asserted to be useful in the treatment of bronchitis, asthma, liver and spleen disorders, inflammation, rheumatism, arthritis and gout (28, 29, 30). These therapeutic properties of the celery seed extracts are generally attributed to the essential oils in the extracts. These essential oils are in fact the volatile oils that constitute 1% - 2% vol/weight of seed, which can be obtained by steam distillation (31). The composition of the essential oils has been established by gas chromatography as described in McLeod et al (32) and Salzer (33). The main constituents of the oil are the terpene hydrocarbons limonene and β-selinene which together constitute about 80% of the total oil. The remainder is made up of other terpenes and compounds which give the characteristic odour of celery oil e.g. the butyl phthalides (31). Scientific data has demonstrated that both aqueous and 80% ethanol extracts of the celery stalk have anti-inflammatory properties as described in Lewis et al (34) and Al-Hindawi *et al*. (35).

In relation to conventional alcoholic extraction of celery, one method is to extract celery seed with 95% ethanol until all ethanol soluble celery components have been extracted. After filtration to remove particulate material, the soluble components are then used as the therapeutic agent based on the content of volatile oils.

Reference also may be made to Derwent Abstract Accession No. 86-187994 which refers to a dried powder prepared from parsley root having anti-inflammatory properties. FR2611502 refers to a crude extract prepared from celery roots which comprises a powder mixed with a preservative such as starch or acacia gum having anti-inflammatory activity.

Reference may also be made to (1) Nitz *et al*., 1992, Journal of Agricultural Food Chemistry 40 No. 6, (2) Parry (1908), The Chemistry of Essential Oils and Artificial Perfumes, (3) Lund, 1978, Journal of Association of Official Analytical Chemists Inc., 61 No. 5 (1978), (4) Bjeldanes *et al*., Journal of Organic Chemistry 42 No. 13 (1977) and (5) Bjeldanes *et al*., Journal of Food Science 43 No. 1 (1978), all of which make it clear that in relation to celery oil or crude extracts of celery, that the principal essential oils include compounds inclusive of sedanolide, sedanenolide and butylphthalide as well as related components sedadonic anhydrde and sedadonic acid. However, celery oil includes other constituents such as dextrolimonene and sesquiterpenes as well as other components and it is not always possible to calculate a specific dosage response for patients to which celery oil has been administered as a therapeutic agent. Also, it is not possible to monitor the concentrations of individual components of celery oil when used as a therapeutic agent. The use of celery oil as a therapeutic agent also may cause undesirable side effects and is not always effective in use.

## SUMMARY OF THE INVENTION

An object of the invention is therefore to provide therapeutic agent(s) for the treatment of inflammatory diseases as well as diseases, illnesses or complaints caused by uric acid accumulation in the body which are effective in use.

The therapeutic agent(s) of the invention may also be used for treatment of endometriosis, which refers to the growth of cells in the lining of the womb in the body cavity which causes an inflammatory response.

The therapeutic agent of the invention is in the form of a celery seed concentrate which, upon HPLC analysis, has peaks A, B and C described herein in relation to Examples I-VI wherein peak A represents sedanolide, peak B represents butylphthalide and peak C represents sedanenolide.

Preferably the composition contains no alcohol or water.

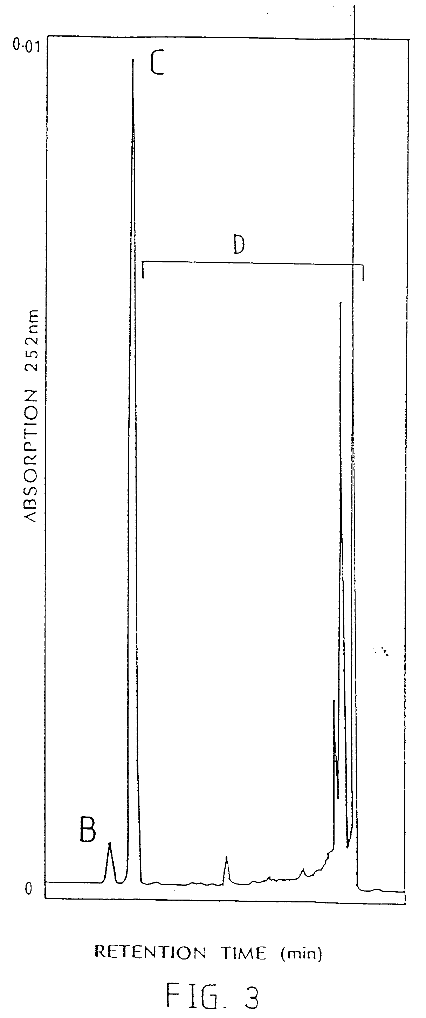

There also may be present in the composition a fourth component which is referred to as component D in FIG. 3 herein.

The celery seed concentrate of the invention may be prepared by a process which includes the steps of:

(i) contacting celery seed with an organic solvent which may extract soluble components of the celery seed to produce a solvent extract;

(ii) separating the mixture of celery seed and solvent so as to remove particulate material therefrom;

(iii) concentrating the extract by removal of the solvent to thereby remove volatile material leaving a residue of non-volatiles; and

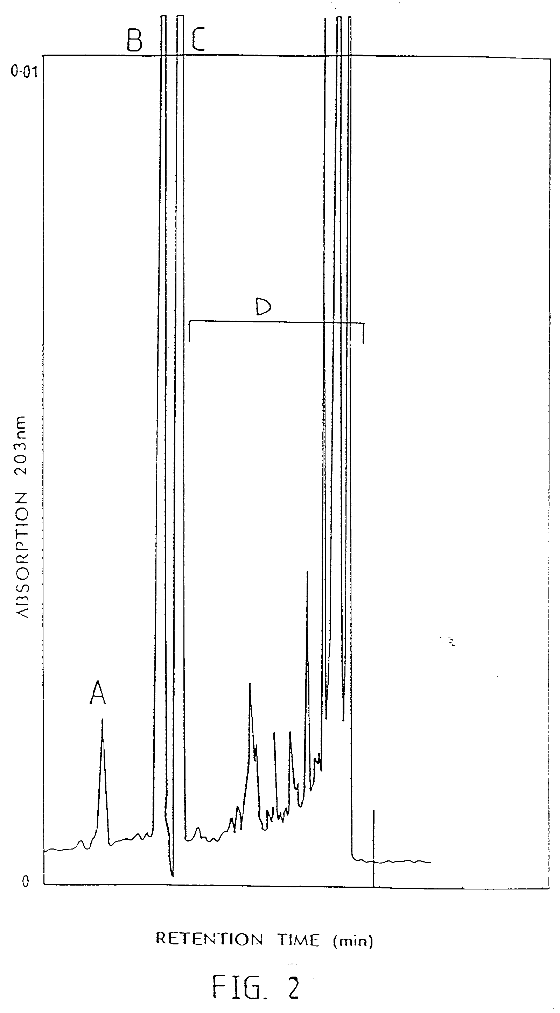

(iv) further concentrating said residue under vacuum to provide a liquid concentrate which after HPLC analysis has peaks A, B and C described herein in FIG 2.

The invention also includes within its scope celery seed extracts prepared in accordance with the abovementioned process.

In relation to celery seed, the varieties of suitable celery seed includes the French, Chinese and Indian species. The Indian variety of celery seed however is preferred.

The celery seed is preferably crushed to a powder. This can be achieved by any one of a number of methods. For a relatively low mass of celery seed, a mortar and pestle is suitable for crushing.

Alternatively, the plant material may be crushed or ground in a ball mill, plate mill or other suitable means for comminuting the plant material. Initially the celery seed which may have an average dimension of 1.5 x 1.55 mm and a thickness of 0.5 mm may be comminuted to provide finely divided particles of an average diameter of 0.1 - 0.2 mm.

Following crushing, the crushed seed may be suspended in a suitable solvent selected from a group comprising alcohol, acetone, methylene chloride, benzene, chloroform, glycols including propylene glycol, ether and appropriate organic oils. Preferably, the solvent is an alcohol. A preferable alcohol is ethanol. A suitable concentration of ethanol is 95% - 98% (v/v). The preferred ratio of seed to ethanol is 1 kg of seed in 16 litres of ethanol but the ratio may be as high as 1 kg of seed in 5 litres of ethanol.

However, in accordance with this invention while it is possible to use alcohol of 95 - 98% concentration it is more effective in the process of this invention to use absolute alcohol or alcohol redistilled from 95 - 98% concentration. Thus the alcohol may have a preferred concentration of 99.99%.

The suspension can be subjected to slow agitation until the extractable components in the celery seed have been fully extracted with the chosen solvent. The temperature of incubation may vary but the preferred temperature is room temperature. Higher incubation temperatures may be used. The incubation may occur in a closed system such as tanks of varying capacities. The incubation time may vary depending upon the celery seed condition but a preferable time may be up to eight days.

Particulate plant material is subsequently removed. Preferably the particulate plant material is removed by filtration and/or sedimentation although centrifugation may also be used. Suitably, the extract is filtered with filters of a pore size that have a range of from 0.2 to 10 microns and more suitably 4-5 microns. Two or more filters maybe used in series. The filtering process may be assisted by the application of positive pressure or the use of a vacuum.

The extract with the particulate plant material removed may then be concentrated to remove solvent and water if present, leaving a residue of non-volatiles. Solvent may be distilled from the extract with the use of heat, vacuum and/or pressure. Where the solvent is ethanol, the temperature of distillation is variable but preferably 50 - 60°C.

In a preferred form of the invention, an initial extract may be prepared which is an alcoholic extract which is a 1 in 10 concentrate (e.g. 1 kg of alcoholic extract is prepared from 10 kg of seed).

The alcoholic extract may be further concentrated under vacuum so that there is a 50% weight loss due to the removal of ethanol and water to provide a viscous liquid or concentrate. Suitably 10 kg of seed provides 0.5 kg of concentrate or a 1 in 20 concentrate.

In accordance with the present invention it is believed that butylphthalide, sedanenolide and sedanolide (in both cis and trans forms otherwise known as essential oils which are mainly derived from plants) mimic the physiological response of essential fatty acids and also their prostaglandin analogues. Such fatty acids comprise the n-6 series derived from linoleic acid and the n-3 series derived from $\alpha$-linolenic acid as discussed in Horrobin and Manku pages 22 - 24 entitled "Clinical Biochemistry of EFA" located in Essential Fatty Acids Pathophysiology and Roles in Clinical Medicine (36).

When the therapeutic agent(s) of the invention are administered to subjects suffering from inflammatory complaints the ingestion or injection of the abovementioned compounds result in an elevation of the same or similar fatty acids in the blood resulting in a reduction of traumatic pain. Essential fatty acids are involved in the regulation of all body functions.

The plant extract of the invention will suitably be marketed in the form of a powder which may be dried under vacuum and which may also contain dicalcium phosphate or other suitable fillers.

A suitable composition for use in the invention may include 5 - 95% by weight of filler. More preferably the filler is included in said composition on a basis of at least 50% by weight and most preferably the filler is included in said composition of at least 75% by weight. In relation to the fillers that may be used in combination with the therapeutic agent of the invention, such fillers may include silica, dextrans, glucose or other suitable carbohydrate. However, dicalcium phosphate may be preferred.

Spectrophotometry of Ethanol Extract

Essential oils are soluble in alcohol and absorb in the U.V. region of 190 - 214 nm (37). Therefore in order to demonstrate the presence of essential oils in the alcohol extract U.V. spectrophotometry was carried out.

Example I demonstrates that the maximum absorption of the extract in 80% alcohol (i.e. ethanol) is at 200 nm when diluted 25,000.

High Performance Liquid Chromatography (HPLC) of the Ethanol Extract

Based on the HPLC methodology described in Applications of HPLC in Biochemistry by Fallon A, Booth R F G and Bell L D, pages 193 - 196 (37), Example II demonstrates the presence of three (3) major peaks of essential oils in the extract and their associated profile. The peaks are A, B and C.

Example III describes preparative HPLC conditions for the isolation and purification of the essential oil peaks A, B and C.

From the isolation of the pure compounds the weight was found and hence the concentration of these essential oils in the ethanol extract and concentrate can be determined.

Spectrophotometry of the Essential Oils (A, B and C) (Example IV)

Spectrophotometry of the pure essential oils (A, B and C) in 80% ethanol demonstrated that there were no other absorption peaks in the visible region and only the characteristic U.V. profile as previously shown in Example I. That is, a peak of absorption at 280 nm and maximum absorption at 191 - 192 nm in this pure form (Example IV).

Mass Spectrophotometry Nuclear Magnetic Resonance and Infrared Analysis (Example V)

Mass Spectrophotometry (MS), Nuclear Magnetic Resonance (NMR) and Infrared Spectrophotometry (IR) have identified the three peaks fatty acids/oils as n-butylphthalide, sedanenolide and sedanolide (Example V).

Extract Formulation

From pilot experiments it was determined that an 80% ethanol solution, containing 34 mg dry weight/ml of the concentrated ethanol extract, would be used for biomedical studies for the relief of rheumatic pain.
The HPLC profile of 34 mg/ml of the ethanol extract is shown in Example VI. Thus the ratio of peaks A, B and C to each other can be calculated from their peak heights. Thus

$$B/A = 9.50$$

$$C/A = 26.25$$

$$C/B = 2.76$$

Hence from the ratios of B/A and C/A, the concentration of these peaks in terms of total solids in the extract and in the diluted extract (34 mg/ml) can be calculated.
Thus in terms of the diluted ethanol extract of 34 mg/ml: 1 : 9.5 : 26.25

$$A = \frac{1}{36.75} \times 34 = 1.008 \text{ mg}$$

$$B = \frac{9.5}{36.75} \times 34 = 8.789 \text{ mg}$$

$$C = \frac{26.25}{36.75} \times 34 = \frac{24.286}{34.083} \text{ mg}$$

In Example III, from a known concentration of peak C (peaks A, B and C are similar) the concentration of the fatty acids/oils A, B and C and region D were calculated using the peak heights shown in Example VI.

| Thus | A = | 0.5 mg/ml |
|---|---|---|
| | B = | 7.5 mg/ml |
| | C = | 20.5 mg/ml |
| | | 28.5 mg/ml |
| The "D" region must be 34 - 28.5 mg = 5.5 mg/ml Thus the ratio of A, B, C, D is - 1:15:41:11 | | |

Plasma Fatty Acids

Since many fatty acids are naturally occurring in human blood, samples of blood were taken before and after the ingestion of an 80% ethanol tincture containing 34 mg/ml of extract and in a capsule form containing 68 mg of extract. The results were similar. In both cases the blood level of these three (3) particular fatty acids/oils and other fatty acids were elevated and then decayed over 3 hours to levels less than that at zero time i.e. before the extract was taken. In addition the blood plasma co-chromatographed with the pure fatty acids/oils A, B and C, demonstrating identity between the fatty acids/oils in the blood and those in the extract (Example VII).

Pilot Study

A pilot study was carried using the McGill Pain Questionnaire: 15 volunteers, age 33-83 years with medically diagnosed rheumatic pain for $10 \pm 8.5$ years that was continual or intermittent. The volunteers were assessed at four consecutive 3 weekly intervals, after which they discontinued taking the extract and were allowed to return to the study of their own volition. At 12 weeks, the volunteers showed a statistical decrease in transformed data of usual pain ($t = 4.2$ $p < 0.001$) and present pain ($t = 5.23$, $p < 0.001$). The decrease in pain was time-dose dependent ($r = -0.90$, $0.001 < p < 0.01$). Also usual pain and present pain were negatively correlated ($r = -0.90$, $0.001 < p < 0.01$). Similarly there was a significant statistical decrease in the number of usual and present body areas experiencing pain ($p < 0.001$) which were correlated with the decreases in usual pain ($0.001 < p < 0.01$) and present pain ($0.02 < p < 0.05$). Upon re-entry into the study, the volunteers average pain level was 70% of the original pain level. The time delay for re-entry into the study was 2-57 days. Decreases in usual pain, present pain, usual and present body areas experiencing pain followed the same pattern as the first part of the study. Standard blood biochemistry and haematology was normal.

Dose Response

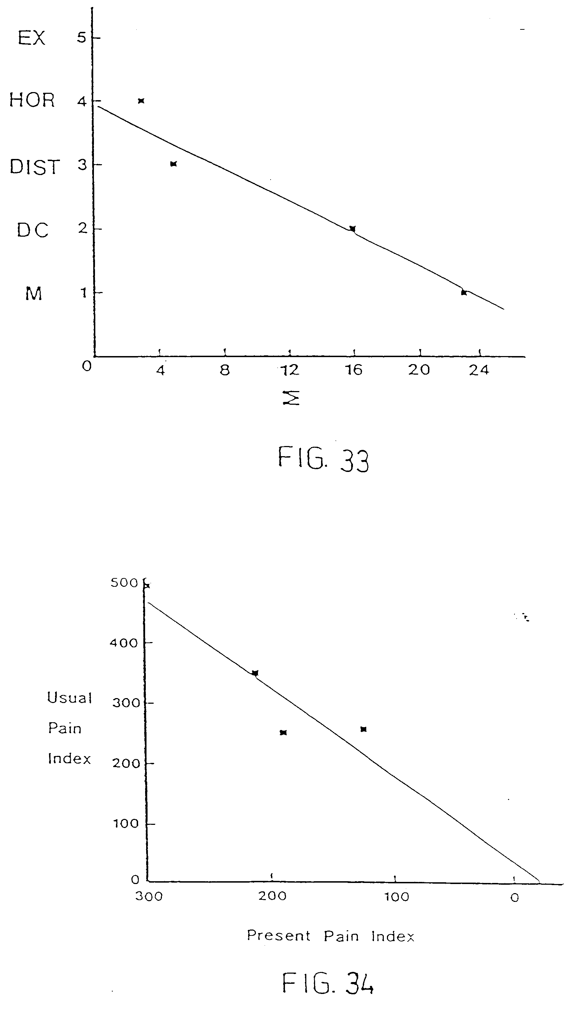

Based on the pilot study of pain relief from arthritic and rheumatic pain, from the correlations previously summarised, and from Example III i.e. present pain is correlated with usual pain and body areas experiencing pain over 12 weeks. Then the amount of extract consumed at 3, 6 and 12 weeks can be plotted against decrease in present pain (FIG. 33). FIG. 34 shows the extrapolation of the graph in terms of extract to be consumed to achieve zero pain.

Thus:

Extract was 34 mg/ml in 80% ethanol, 2 ml/day

= 68 mg of extract/day
= 1428 mg/3 weeks
= 2856 mg/6 weeks
= 5712 mg/12 weeks

Extrapolation gives 11400 mg to achieve zero pain.

1. Over 12 weeks this dose is 958.33 mg/week or 136 mg/day.
2. Thus to achieve zero pain relief in 1 week this dose would be 958.33 x 12 = 11500 mg/week or 1642.85 mg/day or 821.42 mg/bidaily.

This is considered to be the maximum dose per day 1642.85 mg of a 1 in 20 concentrate of celery seed extract.

The minimum dose is that used in this study which is 68 mg/day of a 1 in 20 concentrate of a celery seed extract.

From the Extract Formulation page and Example VI, the ratio of the peaks A, B and C were derived. The concentration of these peaks in an 80% ethanol solution of the extract of 34 mg/ml was then calculated. Thus the same calculations can be carried out for the maximum single dose of 821.42 mg.

| ∴ | A = | 22.35 mg |
|---|---|---|
| | B = | 212.34 mg |
| | C = | <u>586.73</u> mg |
| | | <u>821.42</u> mg |

Similarly as the peaks A, B and C from the pure compounds can be determined Example VI and the D region.

Hence the concentration of the fatty acids/oils A, B, C and D region can be calculated.

| Thus | A = | 12.1 mg/ml |
|------|-----|------------|
| | B = | 181.2 mg/ml |
| | C = | 495.3 mg/ml |
| | | 788.6 mg/ml |
| D region is 821.42 - 788.6 = 32.82 mg/ml. | | |

EXAMPLES

EXAMPLE I

U.V. and Visible Spectrophotometry of the Ethanol Extract

Method

```
Measuring wavelength range . . . . . . . . 900 - 185 nm
Measuring mode . . . . . . . . . . . . . . . . . . (ABS)
Photometric scale . . . . . . . . . . . . . (ABS 0 - 1)
Response time . . . . . . . . . . . . . . . . . . 1 sec
Slit width . . . . . . . . . . . . . . . . . . . 2 nm
Wavelength scale expansion . . . . . . . . . 20 nm/cm
Scan speed . . . . . . . . . . . . . . . . . . . 60 nm
```

(1)      Set absorption cells filled with solvent on the sample and reference sides. (quartz cells used)

(2)      Set up measuring conditions as follows:

```
Start wave length . . . . . . 900 → [GO TO λ]
Measuring mode . . . . . . . ( [ABS]  → Depress)
Response time . . . . . . . . . 1 → [RESPONSE]
Slit width . . . . . . . . . . . . 2 → [SLIT]
Wavelength sale expansion  20 → [CHART FORMAT]
Scan speed . . . . . . . . . 60 → [SCAN SPEED]
Recording mode . . . . . . .  [RECORDING] → OFF
               Set to SEQ for obtaining spectrum
               without background correction.
Light source . . . . . . [LAMP SELECT] → AUTO

Photometric scale   0 → [SCALE]  → 100 → [SCALE]
```

$$(0 \rightarrow \boxed{\text{SCALE}} \rightarrow 1 \rightarrow \boxed{\text{SCALE}})$$

Correcting wavelength . . 900 $\rightarrow \left( \begin{array}{c} \text{BACKGROUND} \\ \text{CORRECTION} \end{array} \right) \rightarrow$ 185 $\rightarrow \left( \begin{array}{c} \text{BACKGROUND} \\ \text{CORRECTION} \end{array} \right)$

ON $(\lambda_1 - \lambda_2)$ nm lamp lights up.

(3)    Depress the SCAN key to start background correction. The UNDER CORR lamp lights up. Background correction is executed in a wavelength range from 900 to 185 nm.

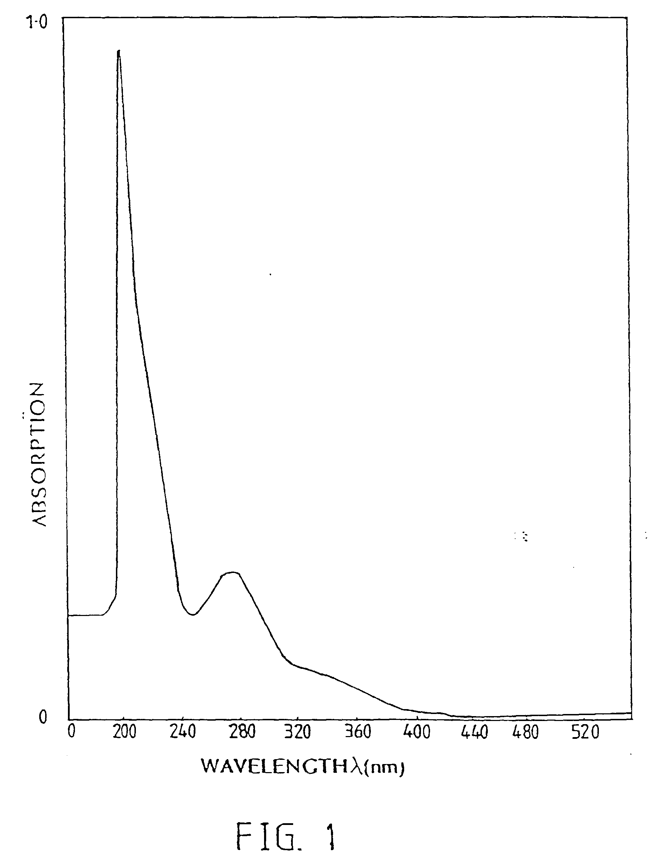

From the resulting visible and UV spectrophotometry diagram as shown in FIG. 1, it was noted that the celery seed extract of the invention had a maximum absorption at 200 nm when diluted 1 in 25,000.

## EXAMPLE II

### High Performance Liquid Chromatography of the Ethanol Extract (Analytical)

H.P.L.C. was carried out in relation to the ethanol extract using a Waterman single pump and a $C_{18}$ analytical column. The buffer was 48% acetonitrile, 52% water, pH 7.0, flow rate 0.5 ml/min, chart speed 5mm/min, absorption 203 nm and a scale of 0.01 full deflection.

FIG. 2 refers to 1$\mu$l of the ethanol extract at a 1 in 10 dilution. The essential oil peaks of interest are A, B and C.

We also attach FIGS. 3 - 5 to demonstrate the absorption range of the essential oils A, B and C.

FIG. 3 refers to 1 in 200 dilution of the ethanol extract showing a loss of peak A at 252 nm and a reduction in the size of peak B.

FIG. 4 refers to a 1 in 50 dilution of the ethanol extract showing a loss of peak A and a reduction in peak B and C at 280 nm.

FIG. 5 refers to a 1 in 50 dilution of the ethanol extract showing the loss of peaks A and B and almost the complete loss of peak C at 340 nm.

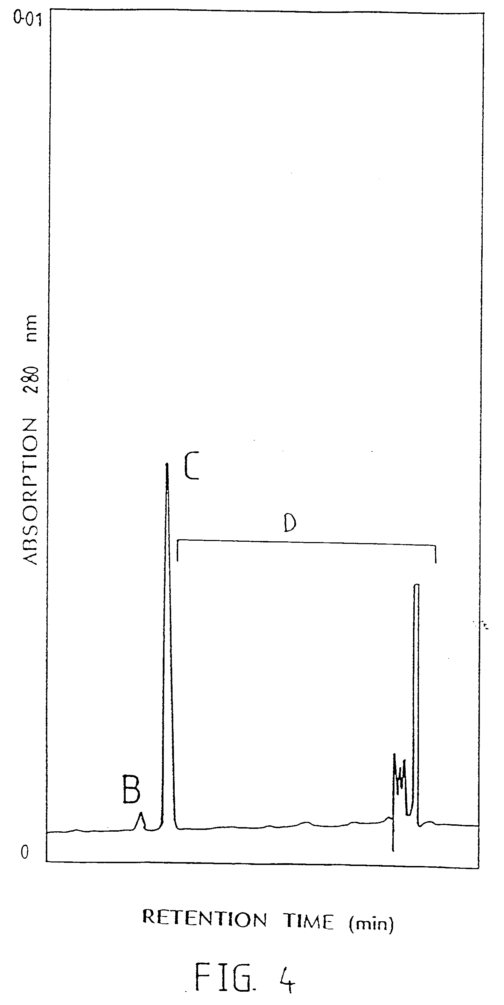

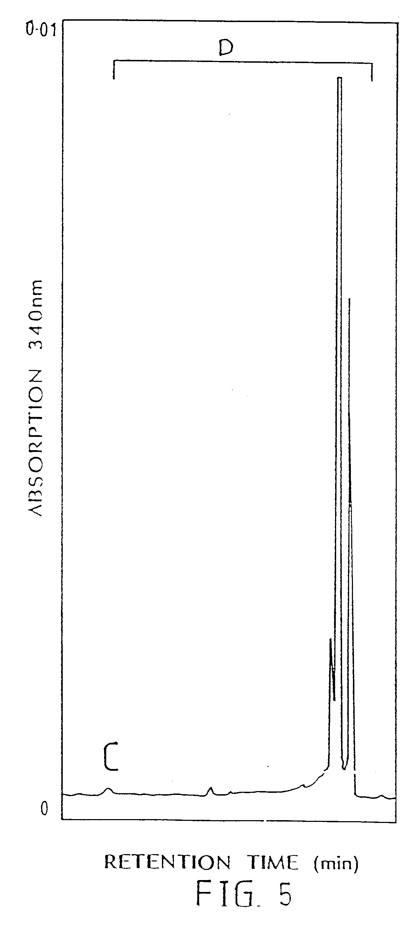

This demonstrates the peaks of the extract in that the absorption U.V. profile (Example II) is due to the essential oils and not to any contaminants in the ethanol extract. D as shown in FIGS. 3-5 corresponds to that part of the absorption trace which does not include peaks A, B and C.

## EXAMPLE III

### High Performance Liquid Chromatography (Preparative)

The extract was diluted 1 in 10 with 48% acetonitrile and 52% water pH 7.0. The resultant black precipitate was then extracted 1 in 20 with hexane. The hexane was removed under vacuum in a rotary evaporator. This gives a green oil. This was then applied to a $C_{18}$ preparative HPLC column using 48% acetonitrile 52% water as buffer.

| | |
|---|---|
| Flow rate | 5 ml/min |
| Scale | 0.4 full deflection |
| Chart speed | 5 mm/min |
| Absorption | 203 nm |

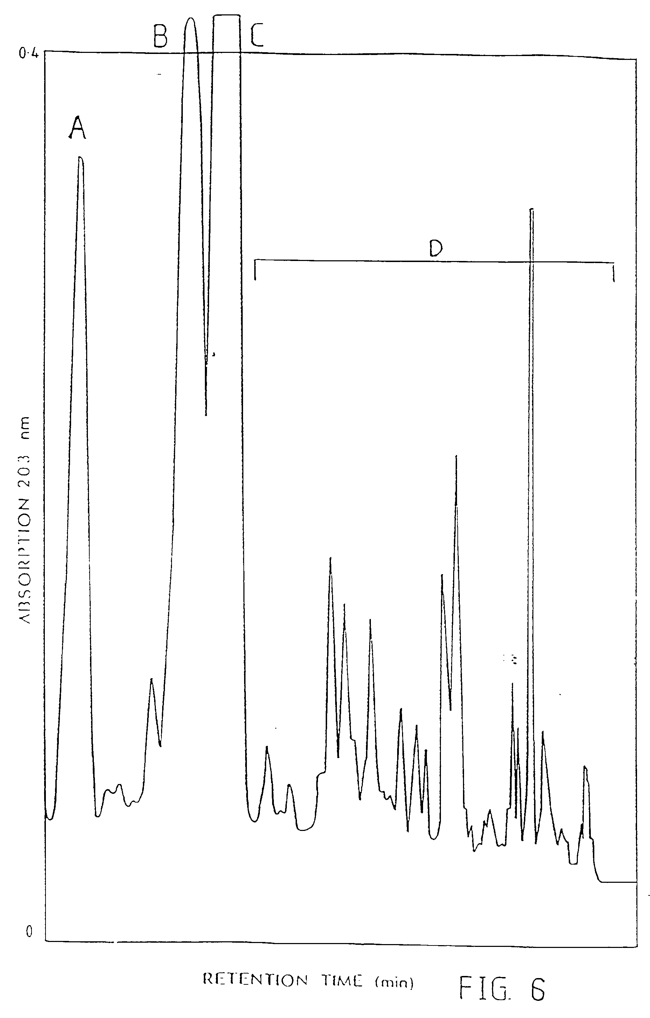

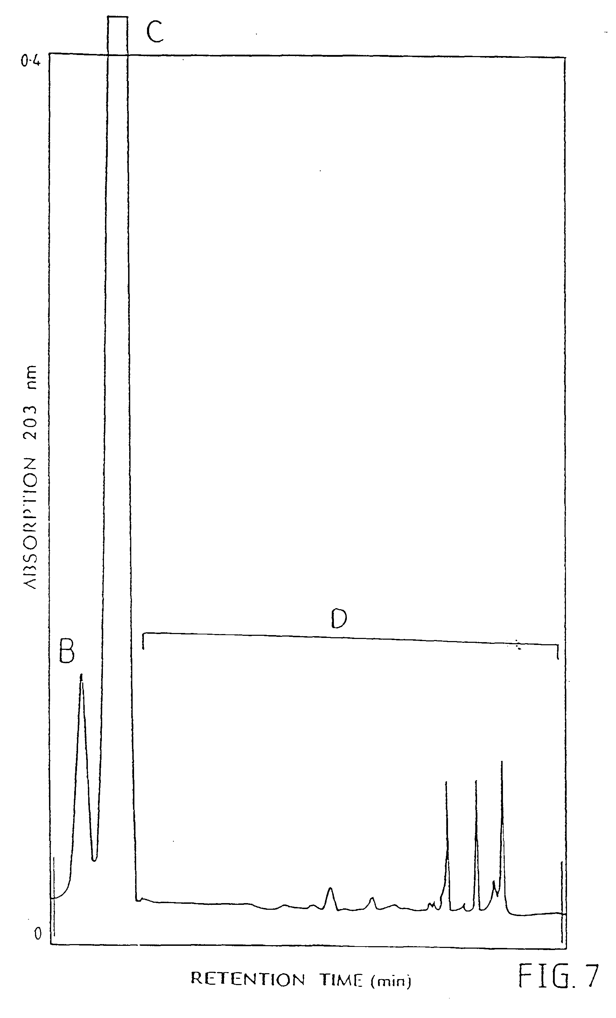

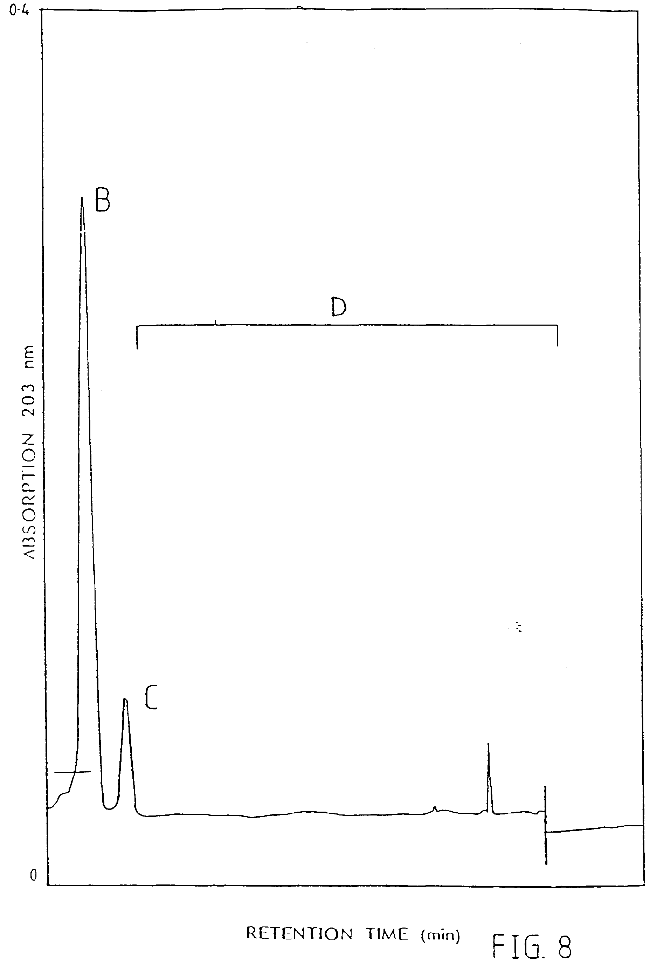

The first isolation of the 3 peaks A, B, C (FIG. 6) demonstrates that peaks B and C would need to be rechromatographed. Thus FIG. 7 shows the rechromatographed peak C and FIG. 8 shows rechromatographed peak B. Thus the three peaks (ABC) have been purified. The solutions of peaks A, B and C were then concentrated under reduced pressure in a rotary evaporator to dryness and weighed and redissolved in 80% ethanol for analytical HPLC as previously described in Example II.

EXAMPLE IV

Spectrophotometry of Fatty Acid Peaks A, B and C

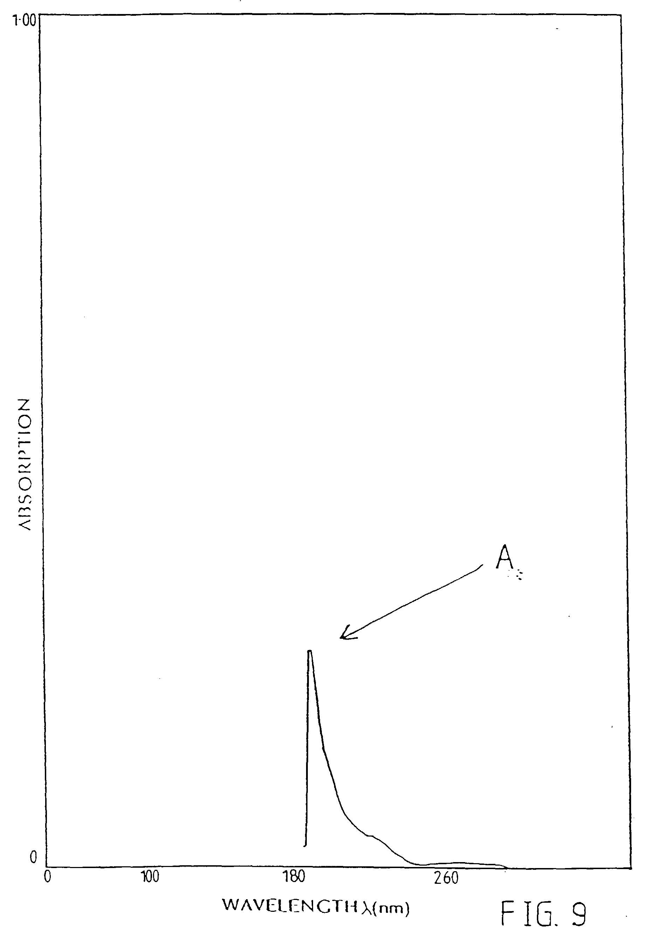

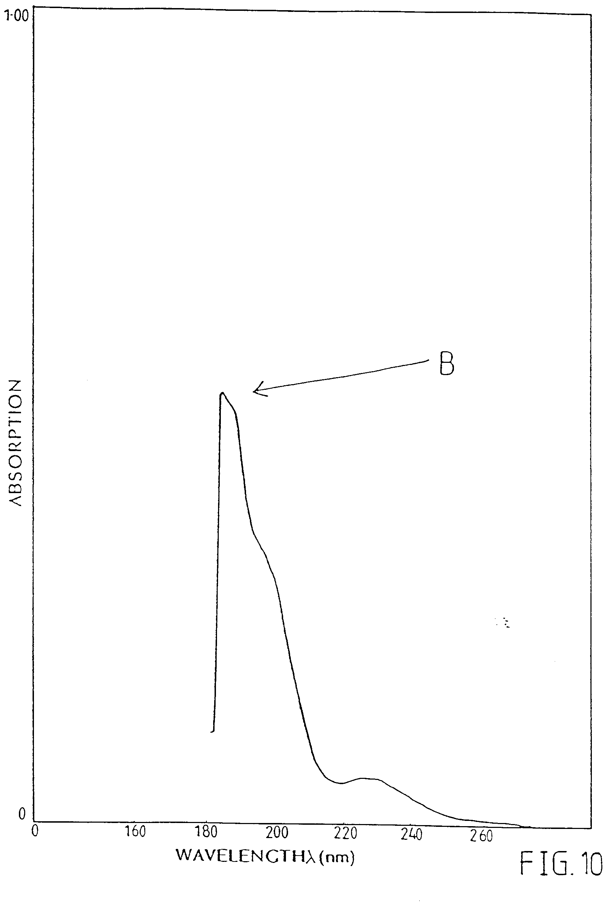

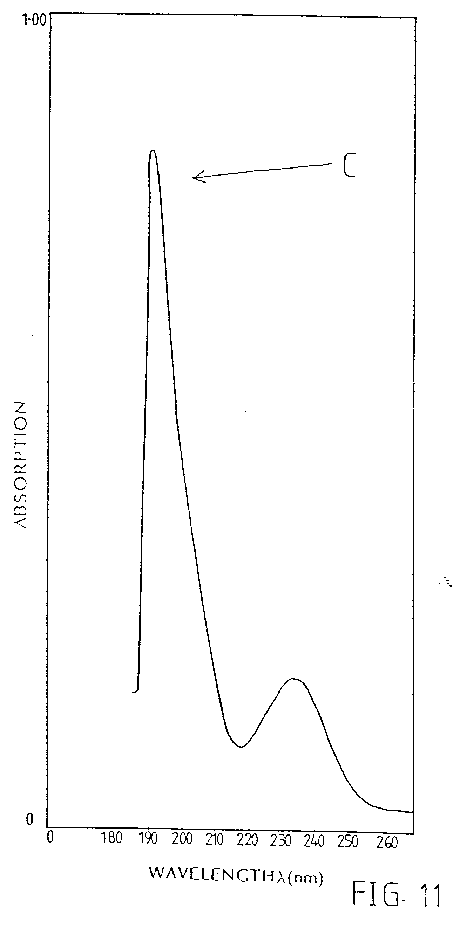

Spectrophotometry in both the visible and U.V. region was carried out as previously described in Example I.
There was no absorption in the visible region of the spectrum. In the U.V. region peak A had maximum absorption at 190 nm peak B 191 nm and peak C 192 nm. UV absorption spectra of peaks A, B and C are shown in FIGS. 9 - 11.

EXAMPLE V

Mass Spectrometry, Nuclear Magnetic Resonance and Infrared Spectrometry

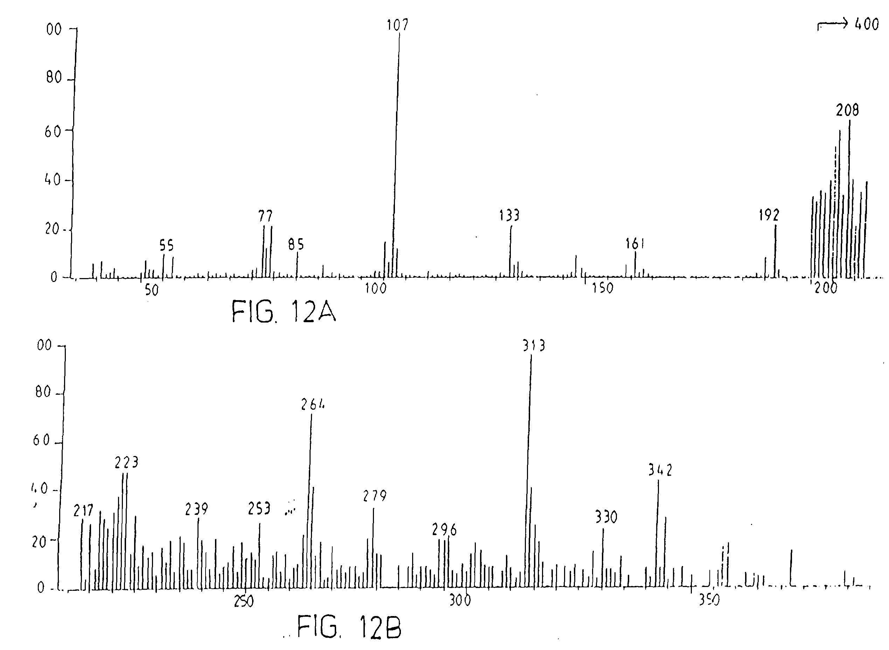

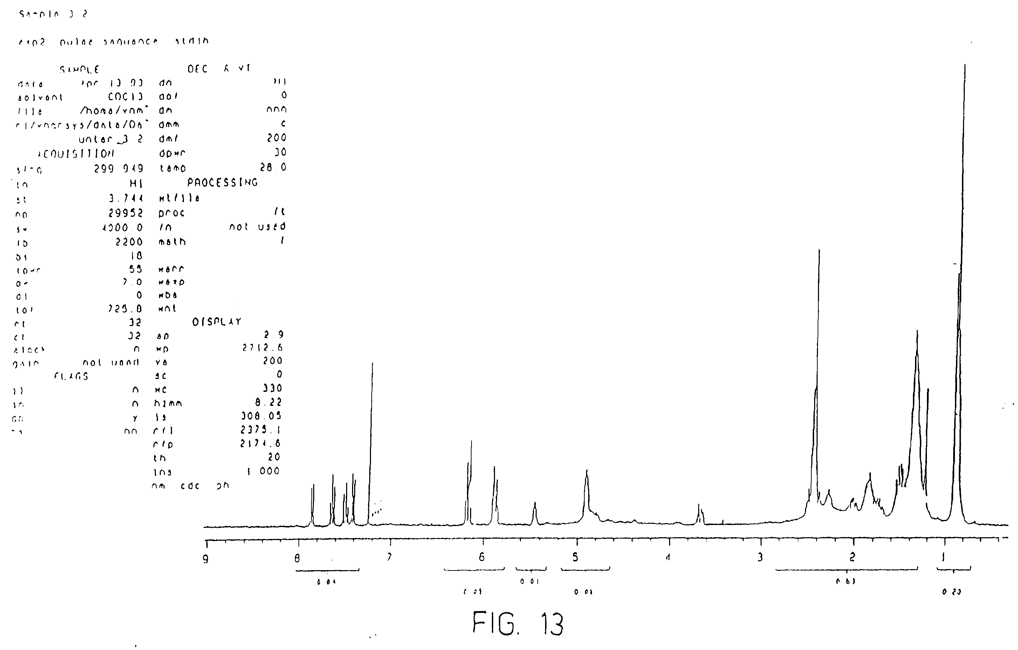

Example V relates to further molecular characterization of the peaks A, B and C previously described. This is shown in FIGS. 12 - 17. More particularly, FIGS. 12 - 17 represent the simultaneous characterization of a sample of peak A, peak B and peak C using Mass Spectrophotometry (MS). Nuclear Magnetic Resonance (NMR) and Infrared Spectrophotometry (IS). FIG. 12 is a MS analysis of peak C. The butylphthalide structure is evident. FIG. 13 illustrates NMR of the same peak C, confirming the conclusions drawn from MS. This shows that approximately 70% sedanenolide and 30% n-butylphthalide are present.

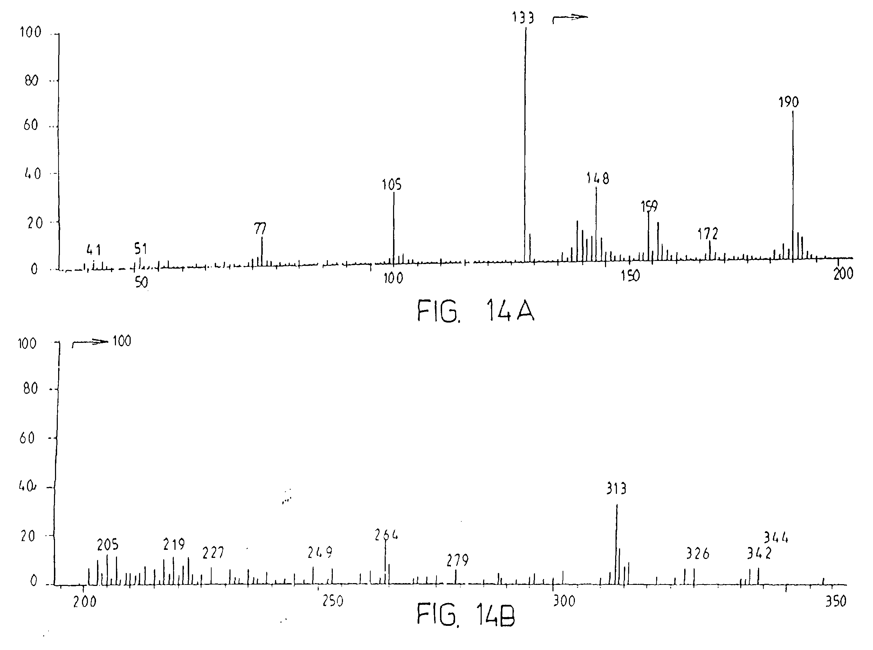

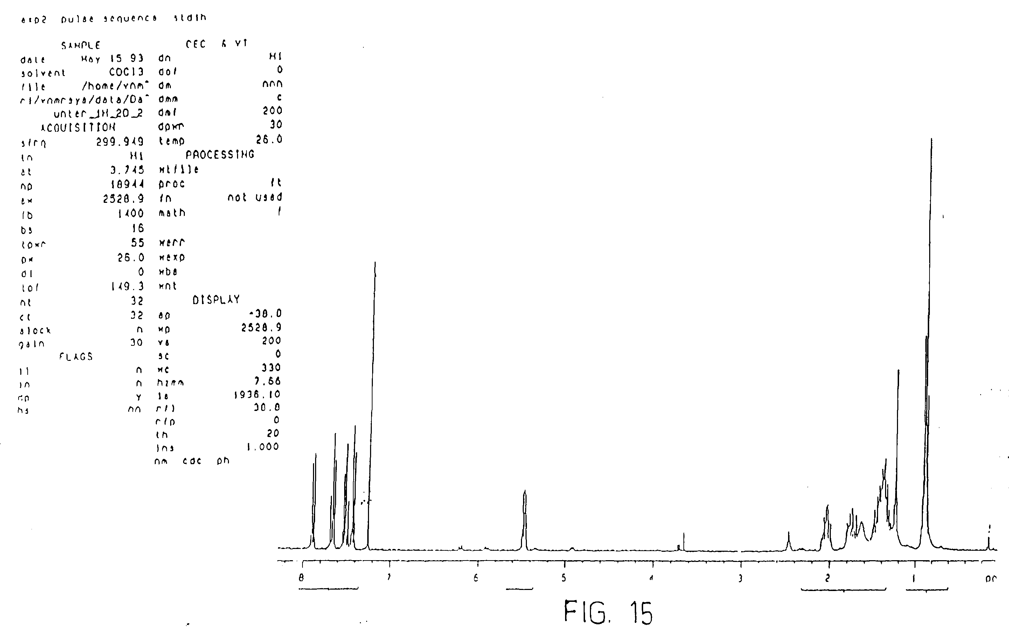

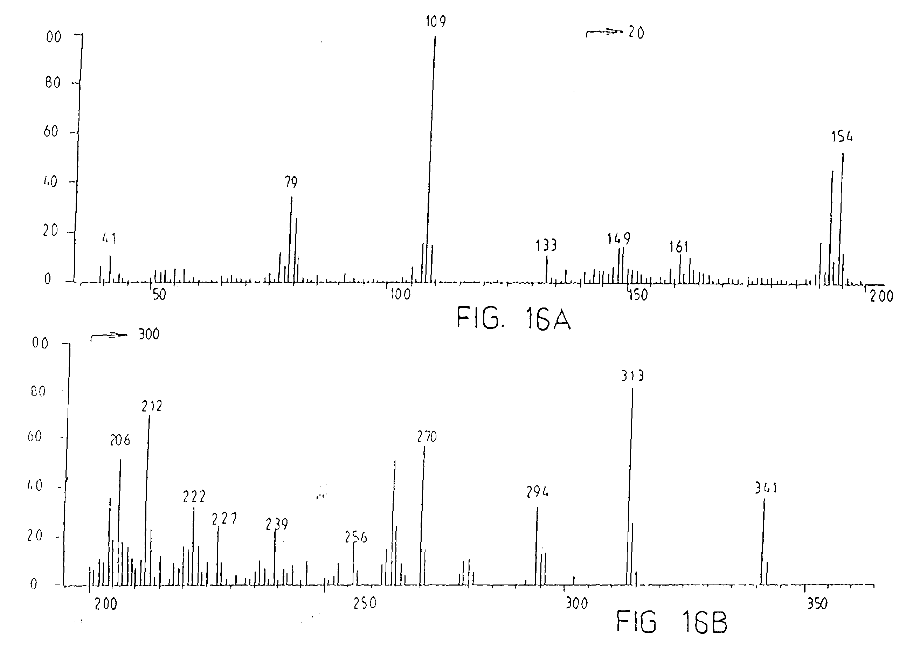

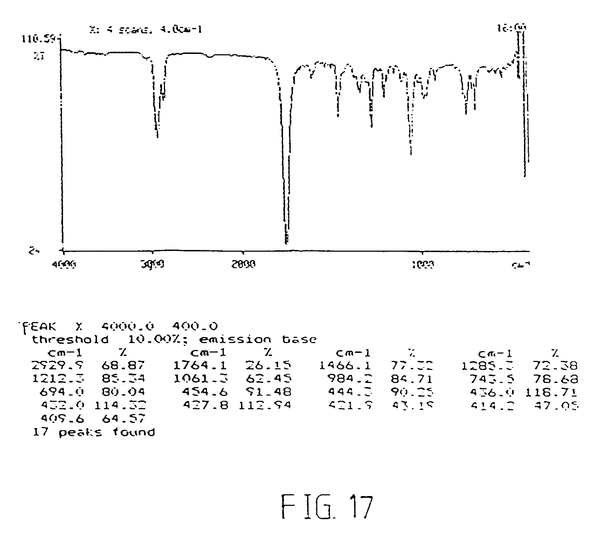

FIG. 14 is a MS analysis of peak B. Again, the butylphthalide structure is evident. This is confirmed in FIG. 15, by the NMR of the same peak B, that shows n-butylphthalide is present. FIG. 16 is a MS analysis of peak A. This Shows that sedanolide is present. Further structural analysis using Infrared Spectrophotometry as shown in FIG. 17 confirms the conclusions derived from the MS.

Analytical HPLC of the Alcohol Extract diluted with 80% Ethanol to give a solids concentration of 34 mg/ml

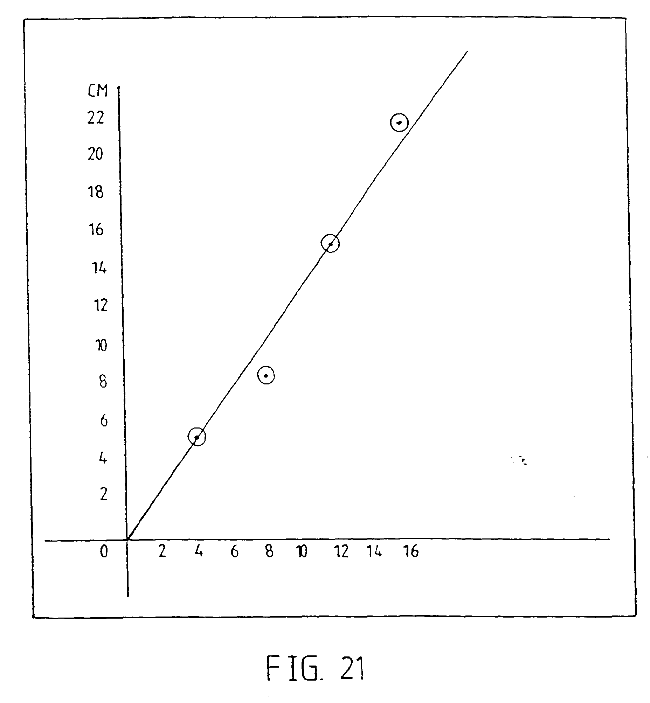

Analytical HPLC was performed as previously described in Example II.

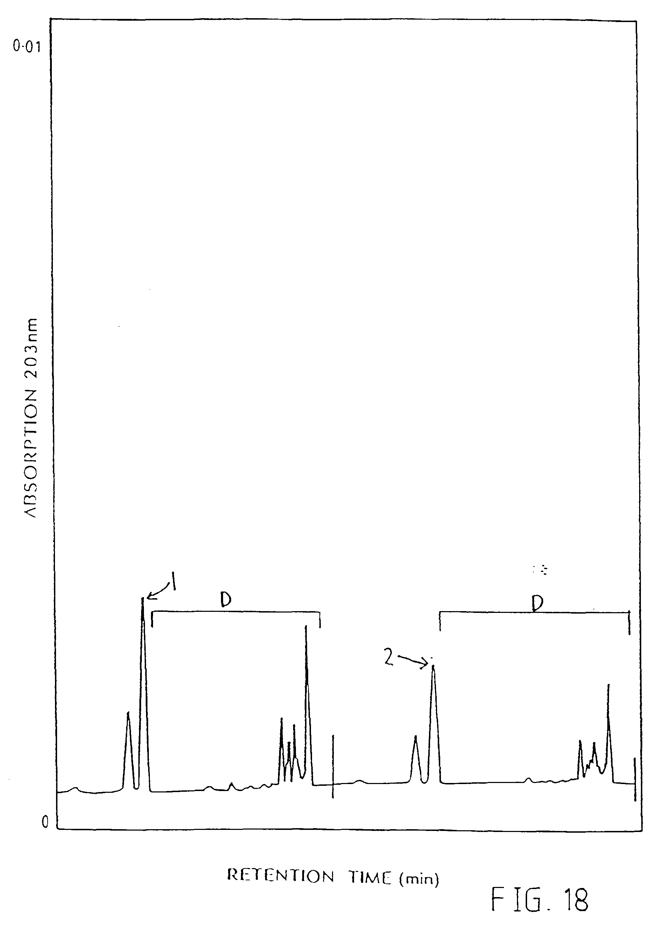

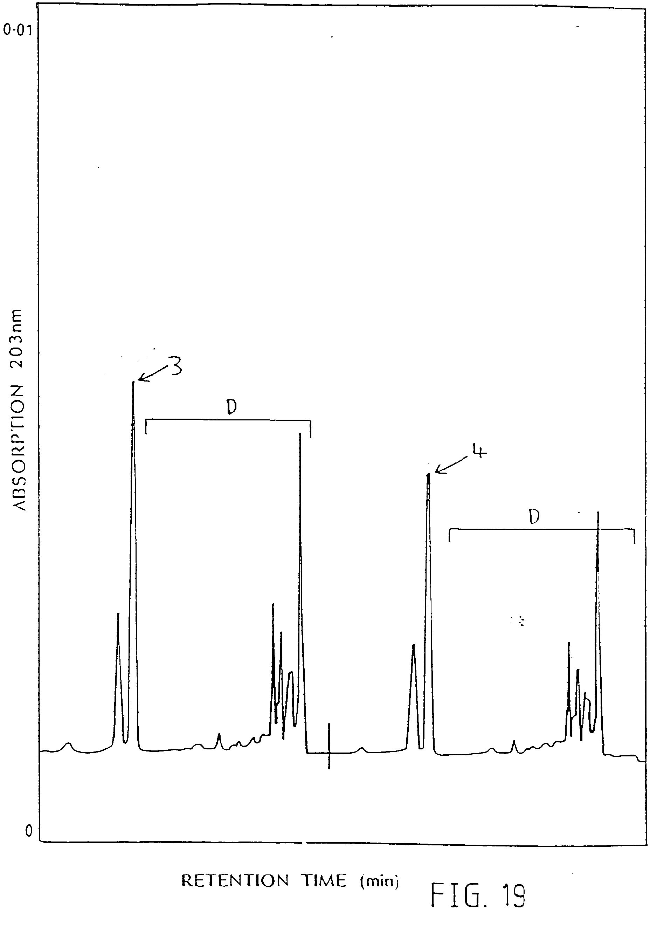

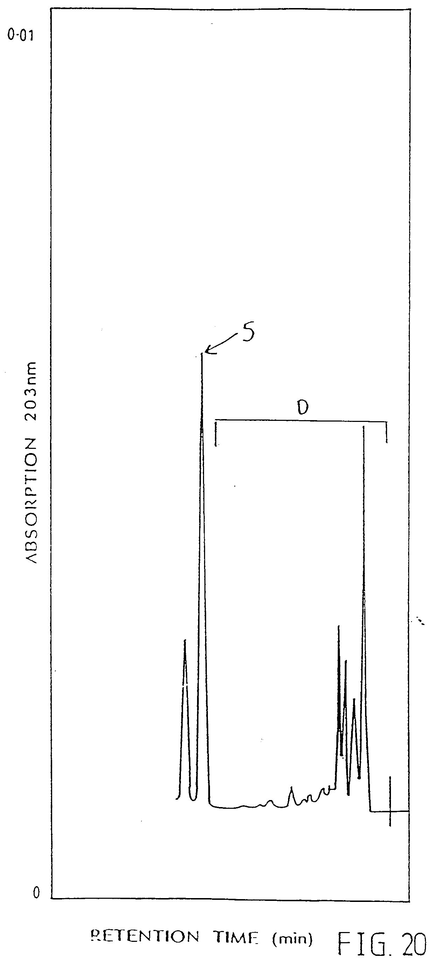

FIGS. 18 - 21 demonstrate the linear relationship of the peak heights to volume of sample used. The sample was the ethanol extract diluted to give 34 mg/ml which was diluted 1 in 10 for HPLC analysis.
Thus in FIGS. 18 - 20 peak 1 corresponds to 2 $\mu$l of the sample, peak 2 corresponds to 1 $\mu$l of the sample, peak 3 corresponds to 4 $\mu$l of the sample, peak 4 corresponds to 3 $\mu$l of the sample and peak 5 corresponds to 5 $\mu$l of the sample.
From this data a calibration curve as shown in FIG. 21 could be constructed and the concentration of the relevant components determined.

EXAMPLE VII

Blood Plasma levels of Fatty Acids before and after ingestion of the Extract

Analytical HPLC was carried out as previously described (Example II).
Blood samples were taken at zero time and at various time intervals thereafter following the ingestion of the extract. Blood samples of 5.0 ml were taken by venipuncture into EDTA-coated tubes and centrifuged to obtain the plasma.
The plasma was then diluted 1 in 2 with 80% ethanol to precipitate proteins and leave fatty acids in solution. A 0.1 ml aliquot of plasma was made up to 0.5 ml with 80% ethanol. Then 0.5 ml of 48% acetonitrile was added, giving a total dilution factor for the plasma of 20. Then 1.0 $\mu$l was taken for analysis.

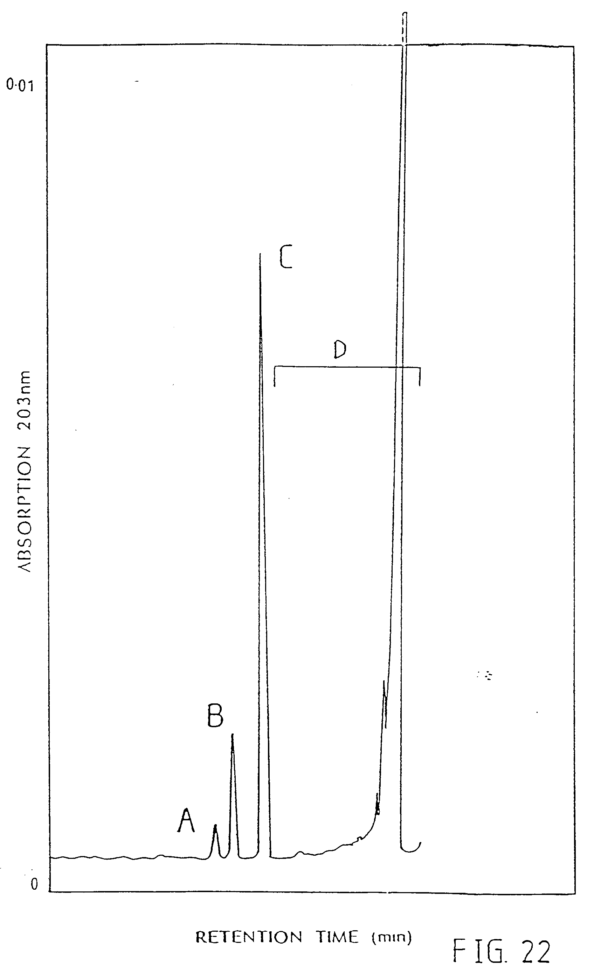

FIG. 22. Subject to zero time showing the presence of the three naturally occurring peaks in the plasma (A,B,C). After taking the ethanol extract (340 mg) the C peak was off scale, hence a 20 fold dilution had to be used.

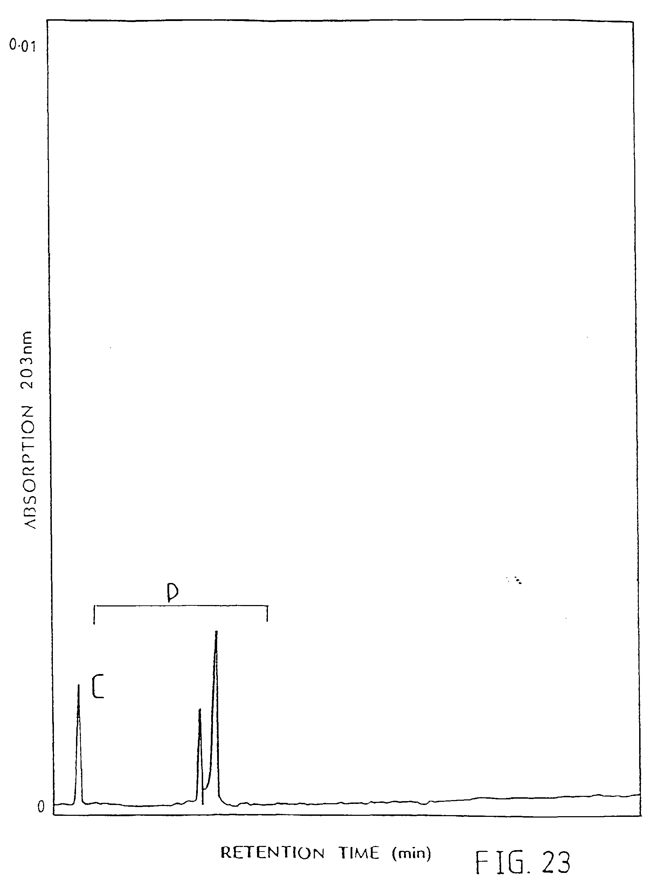

FIG. 23. Subject 1 zero time 20 fold dilution of plasma. Peak C remains but peaks A and B are not detectable at this dilution.

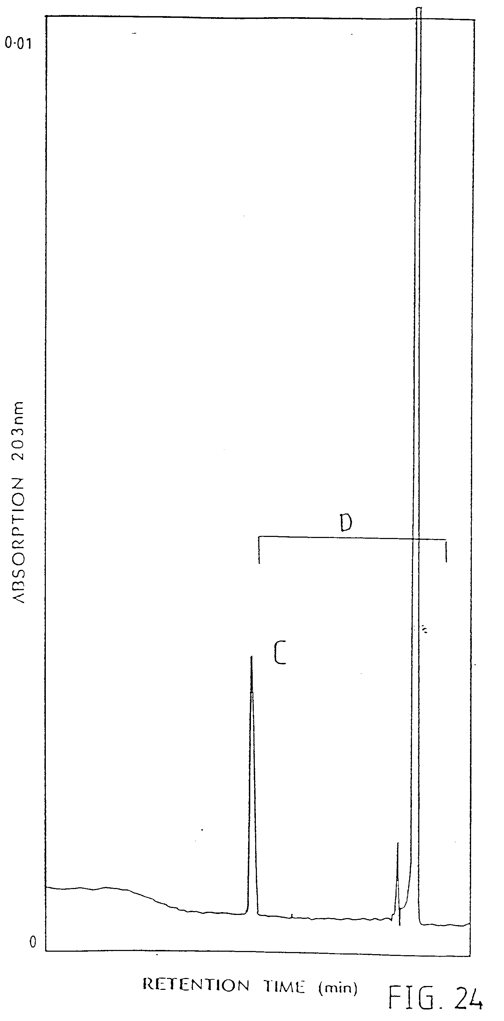

FIG. 24. Subject 1 20 min after ingestion of the extract shows an 108% increase in peak C.

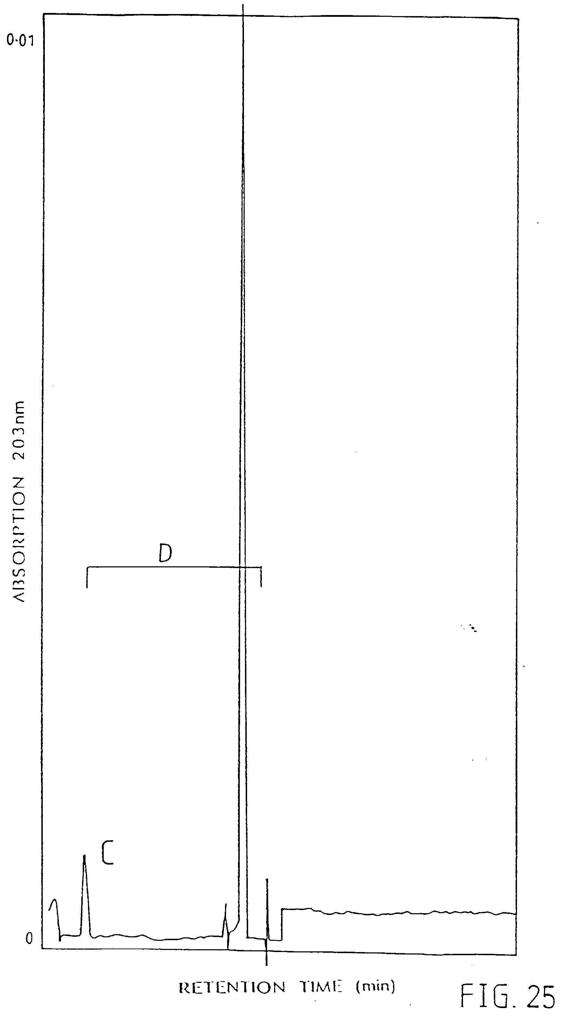

FIG. 25. Subject 1 70 min after ingestion of the extract shows a 33% reduction in peak C.

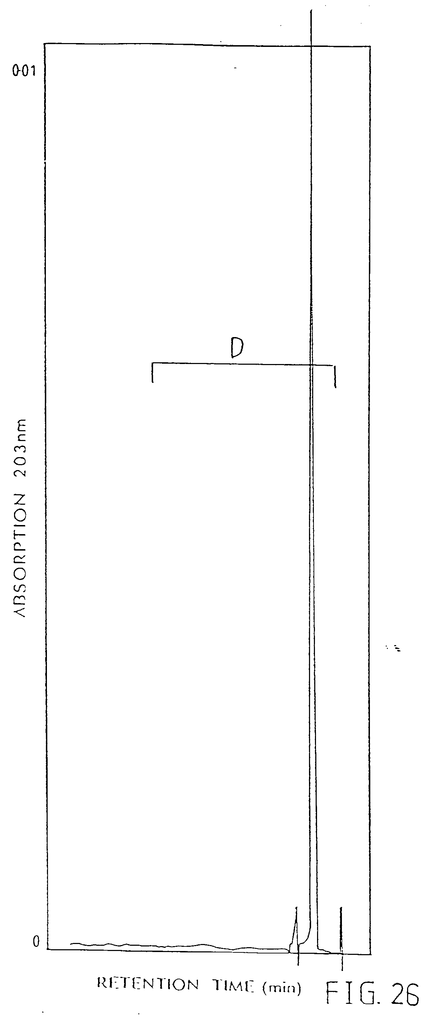

FIG. 26. Subject 1 3 hours after ingestion of extract shows the absence of peak C.

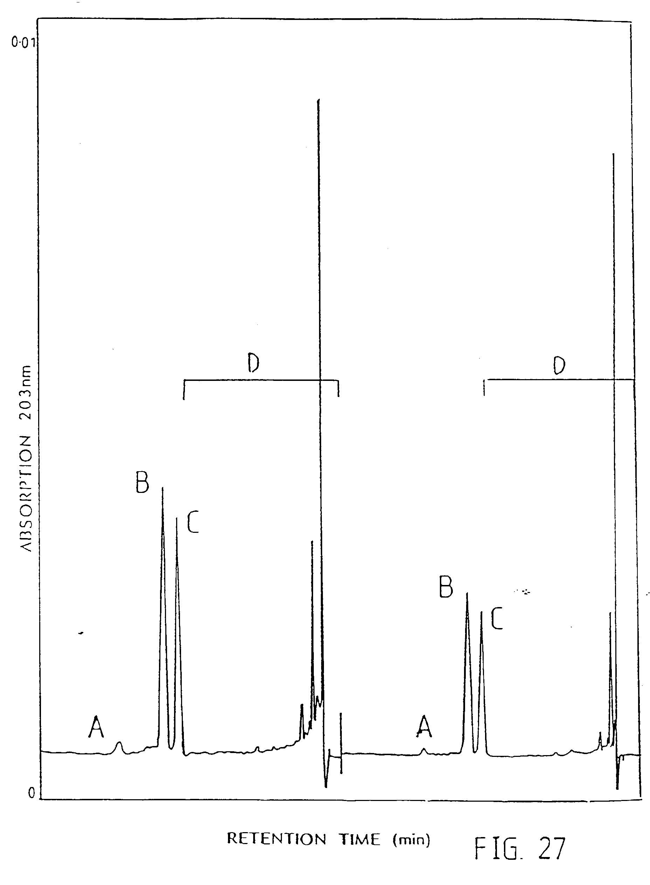

FIG. 27. Subject 2 zero time and 35 min after ingestion of 136 mg of extract. Peak A has increased 50%, Peak B has increased 60% and Peak C has increased 62%.

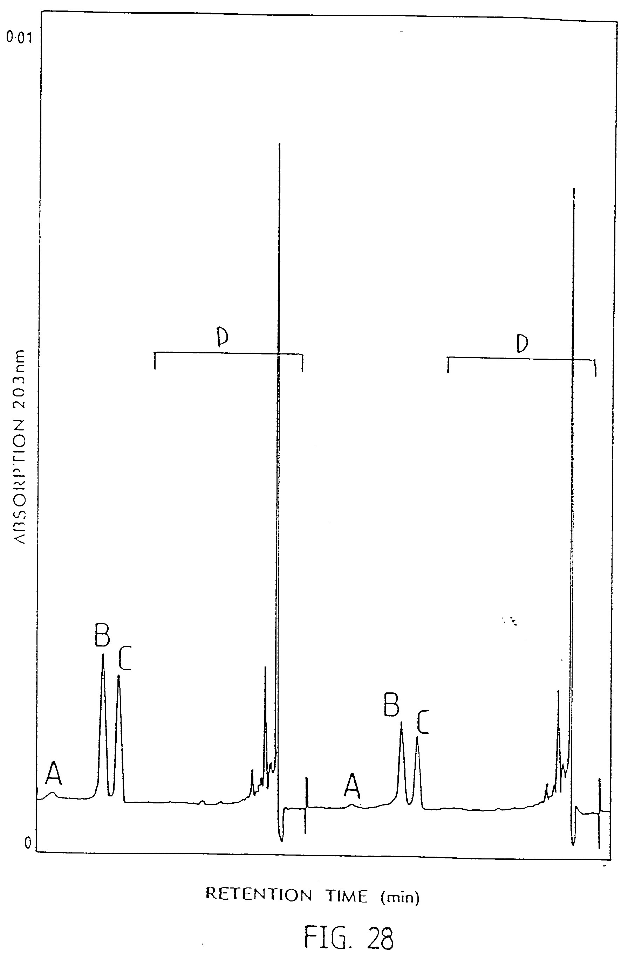

FIG. 28. Subject 3 zero time and 1.0 hours after ingestion of 136 mg of extract. Peak A has increased 50%, Peak B has increased 68% and Peak C has increased 85%.

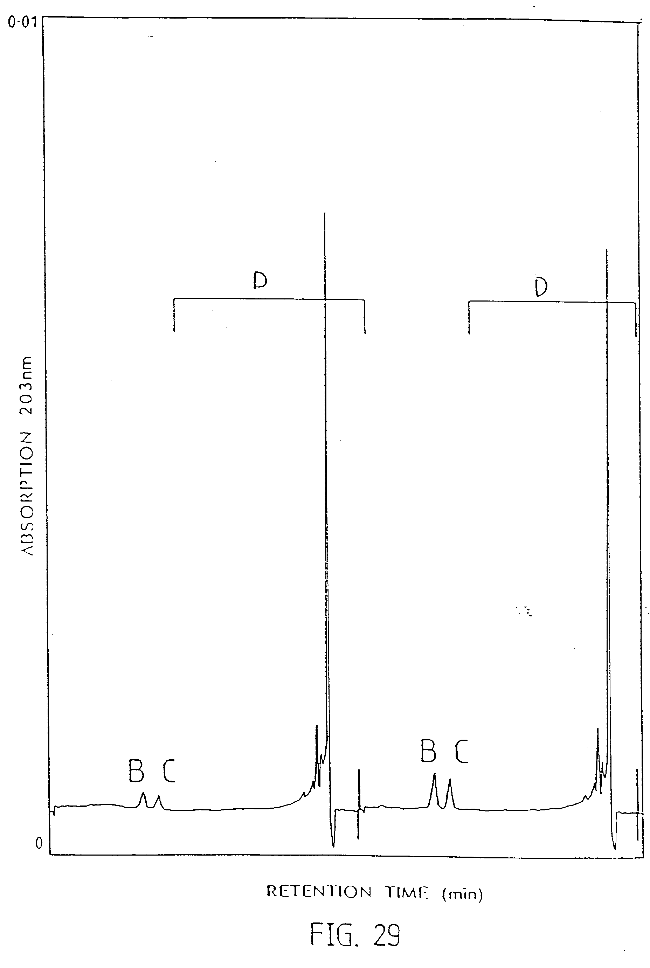

FIG. 29. Subject 4 zero time and 85 min after ingestion of extract. Only Peaks B and C are present which became reduced by 50%.

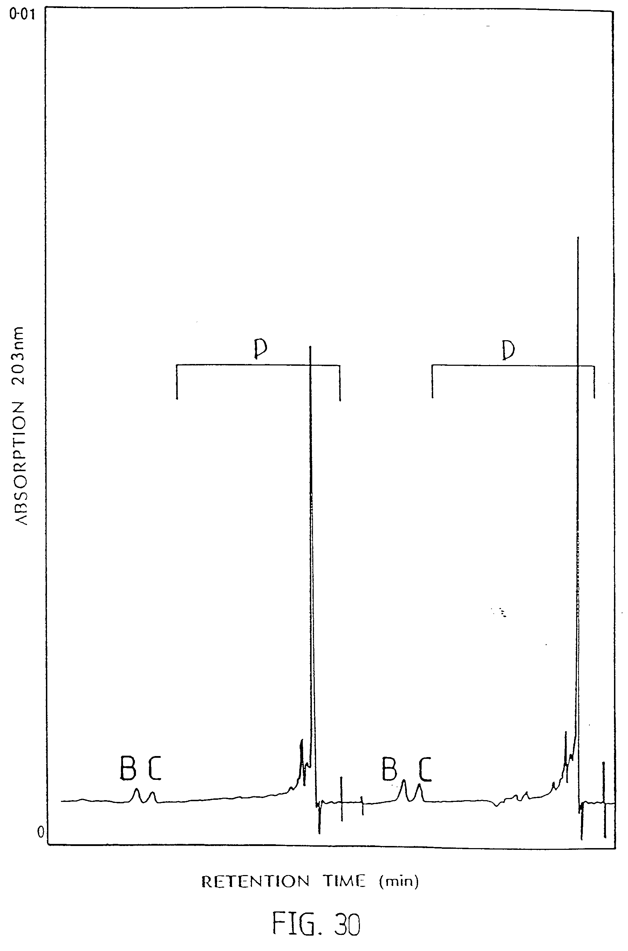

FIG. 30. Subject 5 zero time and 3 hours after ingestion of the extract. Only peaks B and C are present which become reduced by 40%.

These results show that although the zero time concentrations of the peaks A, B and C vary between individuals, they follow the pattern of increase after ingestion of the extract and by 3 hours decrease below that of zero time.

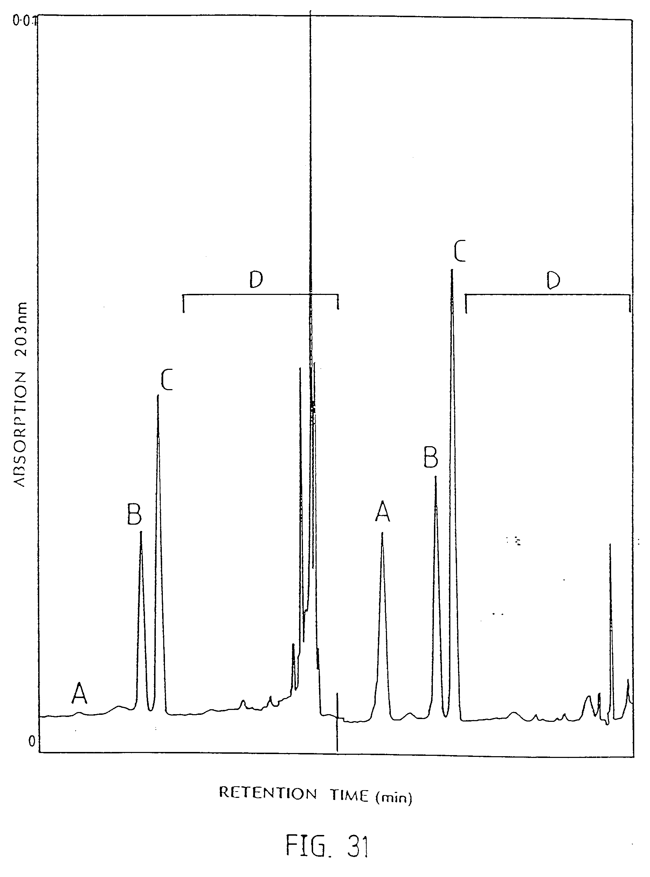

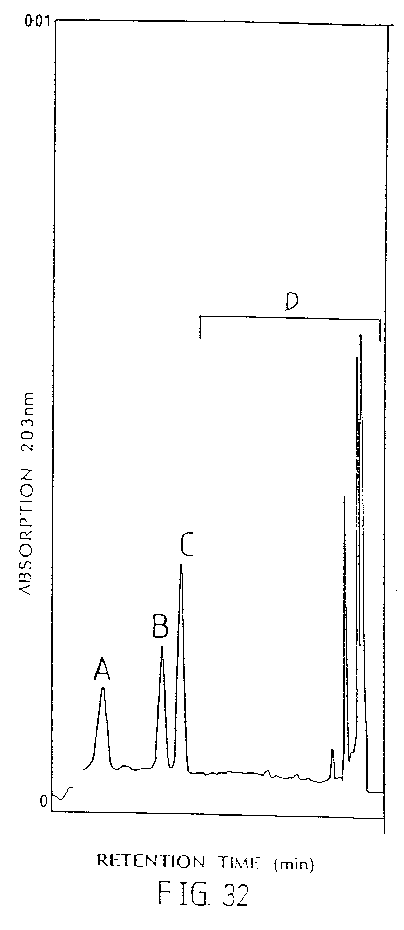

The plasma peaks A, B and C are similar if not identical to those in the extract in terms of retention time on a $C_{18}$ column. Also the pure compounds in the extract and plasma FIG. 31, co-chromatographic FIG. 32.

EXAMPLE VIII

Pilot Biomedical Study for pain relief in Rheumatic Pain

1. Statistical Considerations

1.1 Rheumatic Illness

Rheumatism represents over 100 types of rheumatic illnesses and the syndromes can present manifestations of and/or be associated with other diseases. This complexity therefore makes the classification of rheumatic illness, its treatment and diagnosis difficult. Thus diagnosis has been considered as a variable, which has been controlled/allowed for by accepting volunteers into the study diagnosed by individual medical specialists and general medical practitioners and self diagnosis where proof was evident.

1.2 Pain

The major common factor with rheumatic illness is pain from affected body areas. Therefore the standard McGill Pain Questionnaire was used, which is designed to provide quantitative measures of clinical pain, the pain rating index, that can be assessed statistically and for individual comparisons. In addition, the questionnaire was structured such that the same questions were asked in different ways in order to cross-check responses by statistical analysis.

1.3 Placebo Control

Pain is a variable in that it may be remittent or continual. Therefore assessment of usual pain and usual body areas experiencing pain as well as present pain and present body areas experiencing pain at interview was measured. Therefore, if there is an absence or limited placebo effect, then, usual pain and usual body areas experiencing pain should significantly decrease with time and be correlated. That is, a placebo does not act retrospectively. Similarly, a significant decrease in usual pain with time should be correlated with a significant decrease in present pain. Also, a significant decrease in present pain with time should be correlated with a significant decrease in present body areas experiencing pain. In addition, a significant decrease in usual body areas experiencing pain with time should be correlated with a significant decrease in present body areas experiencing pain.

1.4 Controls

Due to the variables discussed in 1.1 and 1.3, a control group of volunteers would be difficult if not impossible to establish. This may be circumvented by volunteers acting as their own control. This is achieved by expressing pain during the study as a ratio to that experienced at the start of the study.

1.5 Efficacy

Efficacy is in essence a measure of a product's performance claims. Thus products may have a low to high efficacy. This means that for low efficacious products, where few subjects respond to treatment, large numbers of subjects are required for the study to define those who will receive the most benefit. With high efficacious products, few subjects are required to demonstrate efficacy.

### 1.6 Study Design

Consideration was given to cross-over studies (one group) or double cross-over (2 groups) whereby the volunteers at one stage take a placebo and at another stage take the active product. However, since a placebo with the same taste as the active product is not known to exist, and the duration of and mode of action of the active component(s) is not known (washout), cross-over points cannot be established. Thus a longitudinal study was designed in which the first part of the study is repeated when volunteers request to return to the study after cessation of taking the extract. In this way, together with the Statistical Considerations (section 3), a placebo effect is controlled and efficacy can be determined.

A total of 15 volunteers were assessed using the McGill pain questionnaire at the start of the study, and a 5ml blood sample was taken for blood biochemistry analysis. The volunteers were then given a 3-week supply of the extract in tincture form ie. in 80% ethanol at 34 mg/ml. One (1.0 ml) 2 x day taken in a small amount of water.

At the end of 3 weeks, the volunteers were assessed as previously described and the procedure was repeated for another 3 weeks (total 6 weeks). The volunteers were then given a 6-week supply of the extract and a follow-up assessment was conducted at the end of this 6 weeks (total 12 weeks). The volunteers then stopped taking the ethanol extract, and if they wished to return to the study, they notified the field officer and the first part of the study (2x3 weeks) was repeated.

### 2. Results of 12 weeks (0, 3, 6, 12 weeks)

The cohort is composed of 15 volunteers that in the majority of cases (93%) have been medically diagnosed as having arthritic or rheumatic pain, including osteoarthritis, osteoporosis or gout. The pain has been present for approximately 10 years in a remittent or continual form, and the majority (73%) of the volunteers have attempted to use various methods to achieve pain relief. The pain and lack of joint mobility has prevented the carrying out of household duties, hobbies and activities involved in employment.

The general perception of pain by the volunteers is similar to that experienced with rheumatic/arthritic pain. This indicates that these volunteers have had experience of pain over a long duration.

The mean age of the volunteers places them in the higher incidence group for arthritic rheumatic disorders. The current medication, if applicable, for each of the volunteers is shown in Table 1.

**Interpretation**

The correlation between present pain intensity and the sum total ($\Sigma$) of volunteers over 12 weeks as shown in Table 2 and FIG. 31 is statistically significant. This would occur by chance in less than 1 in 100 cases. This demonstrates that present pain intensity for the volunteers continues to decrease over 12 weeks using the ethanol extract (time dependent efficacy).

The statistical analysis is biased against the efficacy of the ethanol extract in that volunteers without pain have not been considered.

**Interpretation**

In relation to interpretation of the data shown in FIG. 34 and Table 3, there was a significant decrease in the total usual pain index at 3 weeks and 6 weeks relative to each other and week 0, whereas at 12 weeks there was no further decrease.

Present pain also showed a significant decrease at 3 weeks which was similar to that at 6 weeks with a further significant decrease at 12 weeks relative to that at 6 weeks. The probability that this would occur by chance alone is less than 1 in 1000 cases.

There is also a significant correlation between the decrease in usual and present pain index. Thus as usual pain decreases over 12 weeks, so does present pain. This would occur by chance alone in less than 5 in 100 cases.

**Interpretation**

The Pain Index for each case at 3, 6 and 12 weeks has been transformed by expressing it as a ratio relative to that at the start of the study, 0 week as described in Table 4. Thus each volunteer is acting as their own control;

$$\frac{\text{Pain Index at 3, 6 or 12 weeks}}{\text{Pain Index at 0 week}}$$

**Interpretation**

In relation to interpretation of the data shown in Table 5, this will be described hereinafter.

**Usual Pain**

The parametric analysis by using the 't' test demonstrates that at 3 weeks there are 4 non-responders and that the mean transformed pain index is not significantly different from that at week 0. At 6 weeks the non-responders had responded and there was now a significant decrease in the transformed mean pain index which is similar to that at 12 weeks. This would occur by chance alone in less than 1 in 1000 cases.

If the non-responders and responders without pain at 3 weeks, and the responders without pain at 12 weeks are not considered, then there is a significant decrease in the mean residual pain index at 3 weeks and 12 weeks. This would occur by chance by less than 1 in 1000 cases.

**Present Pain**

There was a significant decrease in the mean transformed pain index at 3 weeks which is similar to that at 6 weeks and 12 weeks. This would occur by chance in less than 5 in 100 to less than 1 in 1000 cases. If the responders without pain at 3, 6 and 12 weeks are not considered, there is still a significant decrease in the mean residual pain index. This would occur by chance in less than 1 in 100 to less than 1 in 1000 cases.

**Interpretation**

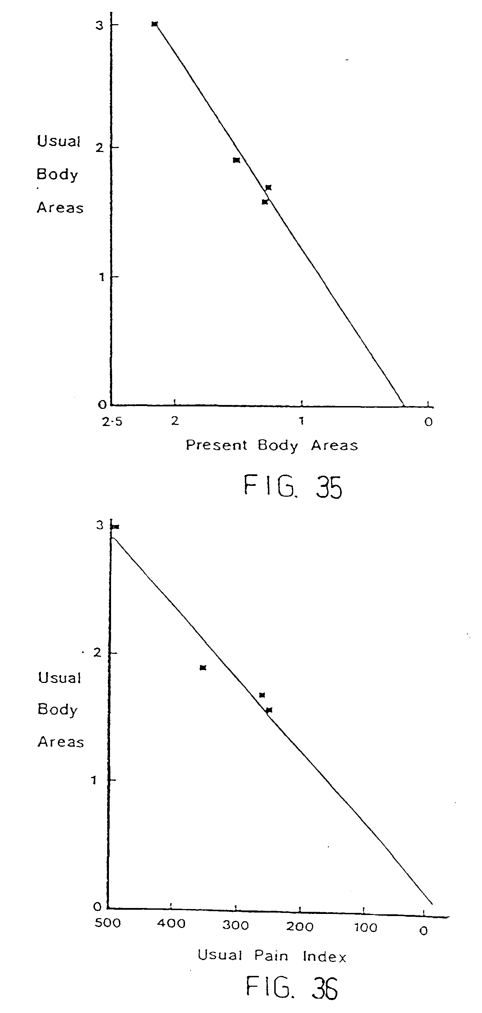

In relation to interpretation of the data shown in FIG. 35 and Table 6, there was a significant decrease in the mean number of usual and present body areas experiencing pain at 3 weeks which was similar to that at 6 and 12 weeks This would occur by chance alone in less than 1 in 1000 cases.

There is also a significant correlation between the decrease in the mean number of usual and present body areas experiencing pain over 12 weeks. This would occur by chance in less than 1 in 1000 cases.

**Interpretation**

In relation to interpretation of the data shown in FIG. 36 and Table 7, the correlation between usual body areas experiencing pain and the usual pain index is significant. This would occur by chance in less than 1 in 100 cases. This demonstrates that as the number of usual body areas experiencing pain decreases, so does the usual pain index decrease. Interpretation

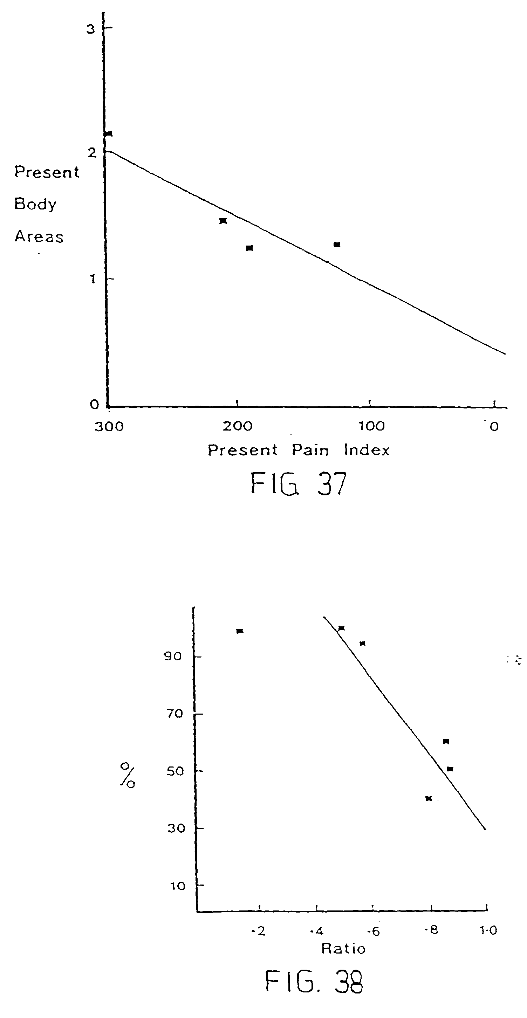

In relation to interpretation of the data shown in FIG. 37 and Table 8, the correlation between present body areas experiencing pain and present pain is significant. This would occur by chance in less than 5 in 100 cases. This demonstrates that as the number of present body areas experiencing pain decreases, so does the present pain index.

3. Follow-up of volunteers upon re-entry to the study after cessation of ethanol extract treatment at 12 weeks

A total of seven volunteers have been lost to follow-up.

Three volunteers discontinued without reason. One volunteer had recurrence of a medical problem and discontinued. One volunteer did not comply because of travelling interstate. Two volunteers were still free of pain at 57 days and did not wish to re-enter the study. By 57 days post-cessation of the ethanol extract treatment, eight volunteers had returned to the study.

After cessation of the ethanol extract at 12 weeks, the volunteers (eight) notified the field officer that they wished to start taking the ethanol extract again. The pain questionnaire was completed and this is referred to as 0 week. The present pain at 0 week was expressed as a percentage of the original present pain (original 0 week at the start of the study). The number of days taken for the pain to increase since cessation of the ethanol extract was noted and expressed as a ratio of the number of days before requesting to start taking the ethanol extract once more. This data is presented in FIG. 38 and Table 9.

**Interpretation**

If the development of present pain becomes approximately equal to the original pain, the ratio is 0.5. That is, the volunteers tolerate pain for an equal amount of time that it took to develop the pain. The lower the percentage of the present pain, relative to that of the original pain at the start-of the study, then the time delay in re-entering the study is greater than the time taken to develop that pain level.

This demonstrates that the request to re-enter the study is not based on a placebo effect.

Mean time for increase in pain $23 \pm 17.4$ days, range 9 - 57 days.

Mean time to re-enter study $34 \pm 15.5$ days, range 9 - 57 days.

Ratio = 0.68.

Thus, on average, volunteers will tolerate 70% of the original pain experience before re-entering the study.

The number of volunteers without pain leaves insufficient data for parametric analysis. Therefore, non-parametric analysis has been performed.

Pain upon re-entry into study

**Interpretation**

In relation to interpretation of the data given in Table 10, the eight volunteers' usual and present paid and usual and present body areas experiencing pain at 12 weeks prior to cessation of the ethanol extract treatment are significantly decreased relative to re-entry into the study (0 week). Thus there has been significant increase in pain and body areas experiencing pain following cessation of the ethanol extract treatment. This would occur by chance alone in less than 5 in 100 to less than 1 in 1000 cases.

Follow-up after re-entry into study

**Interpretation**

In relation to interpretation of the data given in Table 11, there is a significant decrease in usual and present pain at 3 weeks and a further significant decrease at 6 weeks. This would occur by chance in less than 1 in 1000 cases.

There is a significant decrease in usual and present body areas experiencing pain at 3 weeks. This would occur by chance in less than 1 in 1000 cases.

At 6 weeks, there is no further decrease in usual body areas experiencing pain, whereas there is an increase in present body areas experiencing pain, but this is still significantly less ($p < 0.001$) than that at week 0.

The significant decrease in pain and body areas experiencing pain follows the same pattern as that for the first part of the study. The increase at 6 weeks in present body areas experiencing pain, which is still significantly less than that at 0 week, demonstrates the control of breakthrough pain by use of the ethanol extract.

**Interpretation**

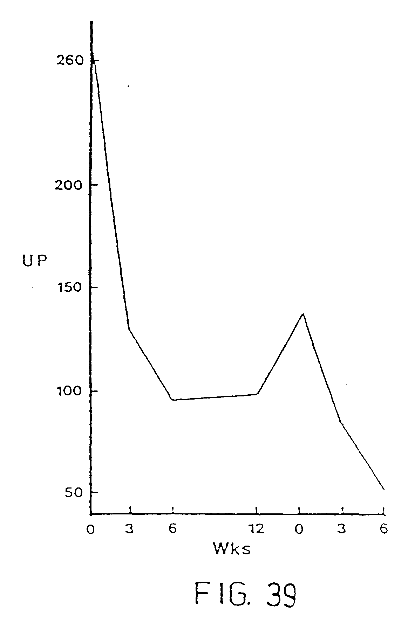

In FIG. 39 and Table 12, there is shown a graphical representation of usual pain of the eight volunteers from the first part of the study (0, 3, 6 and 12 weeks) and re-entry into the study (0, 3 and 6 weeks). This represents the statistical analysis of the McGill Pain Rating Index for usual pain.

**Interpretation**

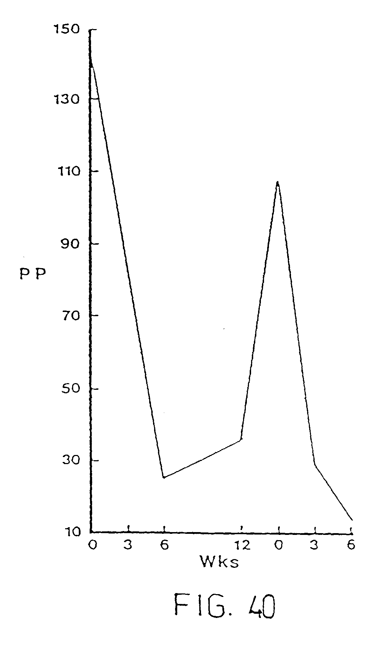

In FIG. 40 and Table 13, there is shown a graphical representation of present pain of the eight volunteers from the first part of the study (0, 3, 6 and 12 weeks) and re-entry into the study (0, 3 and 6 weeks). This represents the statistical analysis of the McGill Pain Rating Index for present pain.

**Interpretation**

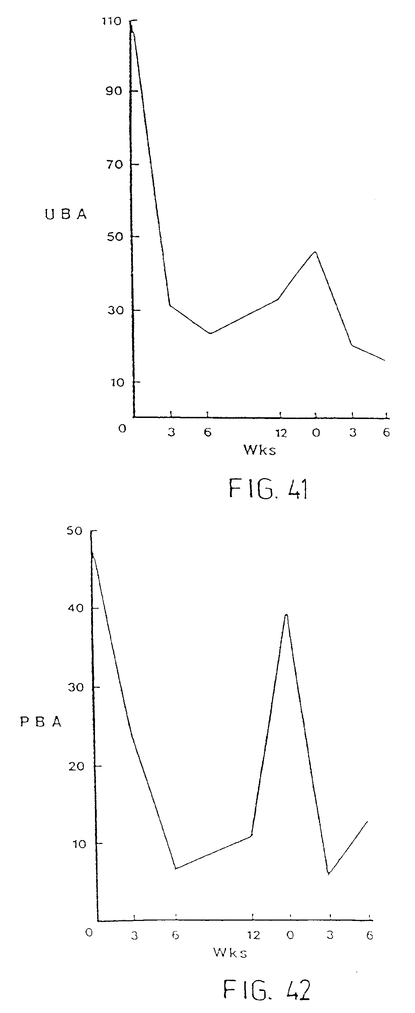

In FIG. 41 and Table 14, there is shown a graphical representation of usual body areas experiencing pain of the eight volunteers from the first part of the study (0, 3, 6 and 12 weeks) and re-entry into the study (0, 3 and 6 weeks). This represents the statistical analysis of the McGill Pain Rating Index for usual body areas experiencing pain.

**Interpretation**

In FIG. 42 and Table 15, there is shown a graphical representation of present body areas experiencing pain of the eight volunteers from the first part of the study (0, 3, 6 and 12 weeks) and re-entry into the study (0, 3 and 6 weeks). This represents the statistical analysis of the McGill Pain Rating Index for present body areas experiencing pain.

4. Blood Biochemistry

In regard to the data shown in Table 16 which represents blood plasma biochemistry analysis, the blood samples were non-fasting and collected in EDTA tubes which limited the scope of the assays. Nevertheless, initial plasma samples can be compared with those samples taken at the time the volunteers were taking the ethanol extract. It can be concluded that the extract does not affect renal or hepatic functions.
One volunteer on initial assessment had elevated levels of glucose, gamma GT, GPT and GOT, which remained elevated at 3, 6, 12 and 18 weeks. Chloride content was decreased at 3, 6, 12 and 18 weeks. The volunteer was consulted and advised to see her doctor. The diagnosis was diabetes and recurrence of sarcoidosis This person was lost to further follow-up.
One volunteer with gout on initial assessment had elevated levels of urea, creatinine and urate, indicating renal dysfunction. These had returned to the normal range at 3 weeks and remained within the normal range at 6 weeks and 12 weeks. This person was lost to further follow-up.

**Interpretation**

In relation to the data shown in Table 17, fasting (8 hours) blood biochemistry and haematology results of seven volunteers who have been taking the ethanol extract for 3 months after the end of the study.

5. Conclusions

The results satisfy the statistical arguments, demonstrating that the ethanol extract reduces pain and body areas experiencing pain for people with rheumatic/arthritic pain.
Although pain relief may occur for some users of the ethanol extract within the first 3-6 weeks of use, the results indicate that pain relief is dependent on the level of pain and the duration of use of the ethanol extract. Thus 12 weeks use of the ethanol extract gives the most efficacious results. Thus from the parametric analysis, the following may be derived.

| 12 WEEKS USE | |
|---|---|
| Usual Pain | mean pain level reduced by 52% range of pain reduction 11% - 100% |
| Usual Pain | residual pain (absence of pain not considered) mean level reduced by 45% range of residual pain reduction 11% - 90% |
| Present Pain | mean level reduced by 68% range of pain reduction 10% - 100% |
| Present Pain | residual pain (absence of pain not considered mean level reduced by 45% range of residual pain 10% - 90% |

Thus since there is no significant difference between the significantly reduced mean values of usual pain and present pain at 12 weeks, it can be stated that the ethanol extract reduces pain on average between 45% - 68% within a range of 10% - 100%. The results also demonstrate that the ethanol extract gives sustained pain relief since there was no breakthrough pain while taking the ethanol extract. This is further demonstrable by the return of pain on cessation of use of the extract and reduction of pain on return to use of the extract.
The pattern of response and the correlation analysis demonstrates that further pain relief would be obtained, for those volunteers with residual pain, if they continue to take the ethanol extract after 12 weeks. Extrapolation indicates a total duration of 22 - 24 weeks would be required.

The ethanol extract does not appear to have any affect on renal and hepatic functions or general blood biochemistry. Similarly there is no effect of the ethanol extract on haematological parameters.

Use of the above composition as a therapeutic agent has the following advantages as will be apparent from the experimental section herein:-

(a) a defined therapeutic response or dosage response can be calculated for each patient;
(b) the concentrations of the individual compounds sedanolide, sedaneolide and butylphthalide in body fluids such as blood may be monitored for topping up purposes;
(c) the therapeutic agent of the invention is very effective in use; and
(d) there are no undesirable side effects after administration to a patient.

REFERENCES

1. Beeson PBN, McDermott W and Wyngaarden JB. (1975). In: Cecil Textbook of Medicine. 5th Edition. Publ. W B Saunders Co., Philadelphia, London, Toronto.
2. Webb J and Nash PT. (1990). Polyarthritis - A diagnostic approach. Australian Family Physician 19(10):1533 - 1542.
3. Kremer JM. (1990). Severe rheumatoid arthritis: Current options in drug therapy. Geriatrics 45(12):43 - 48.
4. Van der Heijde MFM, Van't Hof MA, Van Riel PLCM, Theunisse LAM, Lubberts EW, Van Leeuwen MA, Van Rijswijk MH and Van de Patte LBA. (1990). Judging disease activity in clinical practice in rheumatoid arthritis: first step in the development of a disease activity score. Annals. Rheumatic Diseases 49:916 - 920.
5. Farge D, Liote F, Guillman R and Carpentier A. (1990). Hyperuricemia and gouty arthritis in heart transplant recipients. Amer J Med 88:553.
6. Bomaiaske JS, Williamson PK and Zurier RB. (1983). Prostaglandins and the inflammatory response. Clin Lab Med 3:695.
7. Robinson DF, McGuire MB and Levine L. (1974). Prostaglandins in the rheumatic diseases. Ann N.Y. Acad Sci 256:318.
8. Samealsson B, Dahlem SE, Lindgren JA, Rouzer CA and Serhan CN. (1987). Leukotriene and lipoxins: structure and biosynthesis and biological effects. Science 237:1171.
9. Yoneda T and Mundy GR. Monocytes regulate osteoclast activating factor production by releasing prostaglandins. J Exp Med 150:338.
10. Hasselbacher P and Schumacker HR. (1978). Immunoglobulins in trophi and on the surface of monosodium urate crystals. Arthritis Rheum 21:353.
11. Hasselbacher P. (1979). Binding of IgG and complement protein by monosodium urate crystals and other crystals. J Lab Clin Med 94:532.
12. Wigley FM, Fine IT and Newcombe DS. (1983). The role of the human synovial fibroblast in monosodium urate crystal induced synovitis. J Rheumatol 10:602.
13. Dayer JM, Krane SM, Russell RGG and Robinson DF. (1979) . Production of collagenase and prostaglandins by isolated adherent rheumatoid synovial cells. Proc Natl Acad Sci USA 73:94.
14. McMillian RM, Vater CA, Hasselbacher P, Hahn J and Harris EH. (1981). Induction of collagenase and protaglandin synthesis in synovial fibroblasts treated with monosodium urate crystals. J Pharm Pharmacol 33:382.
15. Rae SE, Davidson EM and Smith JH. (1982). Leukotriene B4 - An inflammatory mediator in gout. Lancet 2:1122.
16. Bomalaski JS, Baker DG, Brophy LM and Clark MA. (1990). Monosodium urate crystals stimulate phospholipase A2 enzyme activities and the synthesis of a phospholipase A2-activating protein. The J Immunol 143:3391 - 3397.
17. Roubenoff R. (1990). Gout and hyperuricemia. Rheumatic Disease Clinics or North America 16(3):539 - 551.
18. Leventhal LJ, Levin RW and Bomalaski JS. (1990). Peripheral arthrocentesis in the workup of acute low back pain. Ach Phy Med Rehabil 71:253 - 254.
19. Hess B and Binswanger U. (1990). Acute uric acid nephropathy in two gouty patients with moderate hyperuricemia and high urine acidity. Klin, Wochenschr 68:874 - 879.
20. Kirwan JR, de Saintonge CDM, Joyce CRB and Currey HLF. (1983). Clinical judgment in rheumatoid arthritis I. Rheumatologists opinion and the development of paper patients. Ann Rheum Dis 42:644 - 647.
21. Kirwan (1983). Clinical judgment in rheumatoid arthritis II. Judging current disease activity in clinical practice.

Ann Rheum Dis 42:648 - 651.

22. Kirwan JR, de Saintonge CDM, Joyce CRB and Currey HLF. (1986). Inability of rheumatologists to describe their true policies for assessing rheumatoid arthritis. Ann Rheum Dis 45:156 - 161.

23. Sany, Clinical and Experimental Rheumatology 8 (Suppl. 5) 81 - 88 (1990).

24. Kumar *et al.*, JAP1 (1990) Vol 38 No. 6 400 - 402.

25. Foreman *et al.*, (1990). Child Nephrol Urol 10:115 - 118.

26. Curtin Microbiological Sciences 1 No. 5 (1984).

27. Webb *et al.* Australian Family Physician 19 No. 10 October 1990, 1533 - 1542.

28. British Pharmacopoeia (1934).

29. Kohli *et al.*, Oct 1967. Indian Jour Med Res 55:1099 - 1102.

30. Kulshrestha *et al.*, 1 Jan 1970. Indian Jour Med Res 58:99 - 102.

31. European Pharmacopoeia Standards.

32. McLeod *et al.*, (1988). Phytochemistry 27 No. 2, 373 - 375.

33. Salzer CRC Critical Reviews in Food Science and Nutrition Nov 1977.

34. Lewis *et al.*, (1985). Int J Crude Drug Res 3 No. 1, 27 - 32.

35. Al-Hindawi *et al.*, (1989). J Ethnopharmacology 26:163 - 168.

36. Price *et al.*, (1987). Critical Reviews in Food Science and Nutrition 26 No. 1.

37. In. Laboratory Techniques in biochemistry and molecular biology. Applications of HPLC in Biochemistry by A. Fallon, R F G Booth and L D Bell. Publ. Elsevier Amsterdam: N.Y. Oxford. Vol. 17. 1990.

TABLE 1

| 1 No medication | 9 |
|---|---|
| 2. Vitamins | 1 |
| 3. Analgesics | 3 (BP or Panadol) |
| 4. Hormones | 1 (Oestrogen) |
| 5. Anti-inflammatory | 1 (Inderel) |

TABLE 2

| | TIME IN WEEKS | | | | |
|---|---|---|---|---|---|
| **Present Pain Intensity** | **0** | **3** | **6** | **12** | **Σ** |
| EX = Excruciating | 0 | 0 | 0 | 0 | 0 |
| HOR = Horrible | 1 | 1 | 0 | 1 | 3 |
| DIST = Distressing | 1 | 2 | 1 | 1 | 5 |
| DC = Discomforting | 7 | 2 | 6 | 1 | 16 |
| M = Mild | 4 | 8 | 3 | 8 | 23 |
| r = -0.972 p > 0.001 < 0.01 | | | | | |

TABLE 3

| Weeks | Σ Usual Pain | $X^2$ | P | Σ Present Pain | $X^2$ | P |
|---|---|---|---|---|---|---|
| 0 | 496.4 | | | 298.7 | | |
| 3 | 354.2 | 40.7 | <0.001 | 212.8 | 24.7 | <0.001 |
| 6 | 252.2 | 29.4 | <0.001 | 189.6 | 2.5 | >0.1 |

TABLE 3 (continued)

| Weeks | Σ Usual Pain | X$^2$ | P | Σ Present Pain | X$^2$ | P |
|---|---|---|---|---|---|---|
| 12 | 261.5 | 0.34 | >0.5 | 125 | 33.4 | <0.001 |
| r = 0.917 p > 0.02 < 0.05 | | | | | | |

TABLE 4

| McGILL PAIN RATING INDEX | | | | | | |
|---|---|---|---|---|---|---|
| **Usual Pain Control 1 ± 0.33** | | | **Present Pain Control 1 ± 0.36** | | | |
| Case | 3 weeks | 6 weeks | 12 weeks | 3 weeks | 6 weeks | 12 weeks |
| 1 | 0.38 | 0.60 | 0.68 | 0.47 | 0.10 | 0.56 |
| 2 | 2.27 | 0.57 | 0 | 2.27 | 0.57 | 0 |
| 3 | 0.53 | 0.15 | 0.10 | 0.11 | 0 | 0 |
| 4 | 0 | 0.26 | 0.46 | 0 | 0.26 | 0.46 |
| 5 | 0.30 | 0.08 | 0 | 0.10 | 0 | 0 |
| 6 | 0.60 | 0.40 | 0.28 | 0.14 | 0.40 | 0 |
| 7 | 0.87 | 0.36 | 0.62 | 1.3 | 0.57 | - |
| 8 | 1.00 | 0.79 | 0.60 | 0 | 0.15 | 0 |
| 9 | 0.77 | 0.81 | 0.57 | 0.57 | 0.82 | 0.18 |
| 10 | 0.82 | 0.78 | 0.81 | 0.84 | 0.82 | 0.86 |
| 11 | 0.42 | 0.44 | 0.20 | 0.62 | 0 | 0.10 |
| 12 | 1.02 | 0.86 | 0.89 | 1.0 | 0.91 | 0.90 |
| 13 | 1.10 | 0.94 | 0.82 | 1.1 | 1.10 | 0.85 |
| 14 | 0.95 | 0.78 | 0.79 | 0.36 | 0.80 | 0.52 |
| 15 | 0.28 | 0.19 | 0.33 | 0 | 0.06 | 0 |
| X* ± sd | 0.76 ± 0.51 | 0.53 ± 0.28 | 0.48 ± 0.30 | 0.59 ± 0.61 | 0.43 ± 0.37 | 0.32 ± 0.34 |
| X** ± sd | 0.53 ± 0.28 | | 0.55 ± 0.25 | 0.40 ± 0.25 | 0.50 ± 0.30 | 0.55 ± 0.29 |

\* X ± sd = total observations

\*\* X ± sd = total observations minus observations > 1 and zero

TABLE 5

| Usual Pain Index | 3 Weeks | | 6 Weeks | | 12 weeks | |
|---|---|---|---|---|---|---|
| | 't' | P | 't' | P | 't' | P |
| X ± sd* | 1.53 | >0.05<0.1 | 4.20 | <0.001 | 4.52 | <0.001 |
| X ± sd** | 3.82 | <0.001 | - | - | 4.10 | <0.001 |
| **Present Pain Index** | | | | | | |
| X ± | sd* | 2.24 | >0.02<0.05 | 4.28 | <0.001 | 5.23<0.001 |
| X ± sd** | 3.46 | >0.001 <0.01 | 3.85 | <0.01 | 3.21 | >0.001 <0.01 |

TABLE 6

| Weeks | Usual Body Areas | | 't' | P Body Areas | Present | 't'P |
|---|---|---|---|---|---|---|
| 0 | 3 ± 1.91 n = 77 | | | 2.15 ± 1.2 n = 68 | | |
| 3 | 1.9 ± 1.1 n = 53 | 4.2 | <0.001 | 1.48 ± 0.63 n = 27 | 3.5 | <0.001 |
| 6 | 1.59 ± 0.81 n = 45 | 5.6 | <0.001 | 1.26 ± 0.64 n = 27 | 4.7 | <0.001 |
| 12 | 1.7 ± 0.77 n = 45 | 5.4 | <0.001 | 1.27 ± 0.56 n = 33 | 5.0 | <0.001 |

TABLE 7

| Weeks | Mean Body Areas | Sum Total Pain Index |
|---|---|---|
| | **Usual** | **Usual** |
| 0 | 3 | 496.4 |
| 3 | 1.9 | 354.2 |
| 6 | 1.59 | 252.2 |
| 12 | 1.7 | 261.5 |
| r = 0.973 p > 0.001 < 0.01 | | |

TABLE 8

| Weeks | Mean Body Areas | Sum Total Pain Index |
|---|---|---|
| | Present | Present |
| 0 | 2.15 | 298.7 |
| 3 | 1.48 | 212.2 |
| 6 | 1.26 | 189.6 |
| 12 | 1.27 | 125.0 |
| r = 0.915 p > 0.002 < 0.05 | | |

TABLE 9

| Present Original Present Pain % | Ratio Days Development of Pain/Restarting the Ethanol Extract Ratio |
|---|---|
| 60 | 42/49 = 0.86 |
| 100 | 14/28 = 0.50 |
| 51 | 13/15 = 0.87 |
| 95 | 21/36 = 0.58 |
| 39 | 29/36 = 0.80 |
| 100 | 7/46 = 0.15 |
| r = 0.827 p > 0.02 < 0.05 | |

TABLE 10

| McGILL PAIN RATING INDEX | | | | |
|---|---|---|---|---|
| | Time in Weeks | | | |
| | Week 12 | Week 0 | Week 0 $X^2$ | Week 0 P |
| Usual Pain | 99.3 | 137.5 | 14.7 | <0.001 |
| Present Pain | 36.6 | 116.3 | 186.0 | <0.001 |
| Usual Body Areas | 34 | 47 | 4.97 | >0.02 <0.05 |
| Present Body Areas | 11 | 40 | 76.4 | <0.001 |

TABLE 11

| McGILL PAIN RATING INDEX | | | | | | | |
|---|---|---|---|---|---|---|---|
| Pain | Wk 0 | Wk 3 | Wk 6 | Wk 3 | Wk 6 | Wk 3 | Wk 6 |
| Usual | 137.5 | 85.3 | 60.7 | 19.8 | 7.1 | <0.001 | <0.001 |
| Present | 116.3 | 39.2 | 25.6 | 51.1 | 4.7 | <0.001 | >0.02 <0.05 |
| UBA | 47 | 22 | 19 | 13.3 | 0.4 | <0.001 | 0.5 |
| PBA | 40 | 5 | 13 | 30.6 | 12.8 | <0.001 | <0.001 |

UBA = usual body areas total number
PBA = present body areas total number

TABLE 12

| Time in Weeks (Wks) | Usual Pain (UP) |
|---|---|
| 0 | 262.4 |
| 3 | 130.1 |
| 6 | 97.2 |
| 12 | 99.3 |
| 0 | 137.5 |
| 3 | 85.3 |
| 6 | 52.6 |

TABLE 13

| Time in Weeks (Wks) | Present Pain (PP) |
|---|---|
| 0 | 144.6 |
| 3 | 83.2 |
| 6 | 26.5 |
| 12 | 36.6 |
| 0 | 110.0 |
| 3 | 29.2 |
| 6 | 13.7 |

TABLE 14

| Time in Weeks (Wks) | Usual Body Areas (UBA) |
|---|---|
| 0 | 109 |

TABLE 14 (continued)

| Time in Weeks (Wks) | Usual Body Areas (UBA) |
|---|---|
| 3 | 31 |
| 6 | 24 |
| 12 | 34 |
| 0 | 47 |
| 3 | 20 |
| 6 | 16 |

TABLE 15

| Time in Weeks (Wks) | Present Body Areas (PBA) |
|---|---|
| 0 | 48 |
| 3 | 24 |
| 6 | 7 |
| 12 | 11 |
| 0 | 40 |
| 3 | 6 |
| 6 | 13 |

TABLE 16

| BLOOD BIOCHEMISTRY | | | |
|---|---|---|---|
| Phosphate | Normal | Sodium | Normal |
| Total protein | Normal | Chloride | Normal |
| Albumin | Normal | Bicarbonate | Normal |
| Globulins | Normal | Glucose | Normal |
| Cholesterol | Normal | Total bilirubin | Normal |
| Triglycerides | Normal | Conjugated bilirubin | Normal |
| Urea | Normal | Gamma GT | Normal |
| Creatinine | Normal | GPT | Normal |
| Urate | Normal | GOT | Normal |

TABLE 17

| BLOOD BIOCHEMISTRY | | | |
|---|---|---|---|
| Sodium | Normal | ALT (SGPT) | Normal |
| Potassium | Normal | AST (SGOT) | Normal |
| Chloride | Normal | LDH | Normal |
| Bicarbonate | Normal | Calcium | Normal |
| Other anions | Normal | Adjusted for Albumin | Normal |
| Glucose | Normal | Phosphate | Normal |
| Urea | Normal | Total protein | Normal |
| Creatinine | Normal | Albumin | Normal |
| Urate | Normal | Globulins | Normal |
| Total bilirubin | Normal | Iron | Normal |
| Conjugated bilirubin | Normal | Cholesterol | Normal |
| ALK Phos. | Normal | Triglycerides | Normal |
| Gamma GT | Normal | | Normal |

**Claims**

1. A therapeutic agent for the treatment of an inflammatory complaint in the form of a celery seed concentrate which, upon HPLC analysis, has peaks A, B and C described herein in FIG. 2 wherein peak A represents sedanolide, peak B represents butylphthalide and peak c represents sedanenolide.

2. A therapeutic agent as claimed in claim 1 which contains substantially no alcohol or water.

3. A therapeutic agent as claimed in any preceding claim wherein there is present in said composition a fourth component represented by area D in FIG. 2 herein .

4. A therapeutic agent as claimed in Claim 3 wherein the ratio of sedanolide : butylphthalide : sedanenolide and the fourth component present in said composition is approximately 1:15:41:11.

5. A therapeutic formulation comprising a therapeutic agent as claimed in any preceding claim which also includes a pharmaceutically acceptable filler.

6. A therapeutic formulation as claimed in Claim 5 wherein the filler is included in said formulation on a basis of 5 - 95% by weight.

7. A therapeutic formulation as claimed in Claim 6 wherein said filler is included in said formulation on a basis of 50% or more by weight.

8. A therapeutic formulation as claimed in Claim 7 wherein said filler is included in said formulation on a basis of 75% or more by weight.

9. A therapeutic formulation as claimed in any one of Claims 5 - 8 wherein the filler is dicalcium phosphate.

10. A therapeutic agent as claimed in Claim 2 in the form of a viscous liquid or concentrate.

11. A process of obtaining a celery seed concentrate useful in treatment of inflammatory complaints including the steps of:

(i) contacting celery seed with an organic solvent which may extract soluble components of the celery seed to produce a solvent extract;

(ii) separating the mixture of celery seed and solvent so as to remove particulate material therefrom;

(iii) concentrating the extract by removal of the solvent to thereby remove volatile material leaving a residue of non-volatiles; and

(iv) further concentrating said residue under vacuum to provide a concentrate which after HPLC analysis has peaks A, B and C described herein in FIG 2 wherein peak A represents sedanolide, peak B represents butylphthalide and peak C represents sedanenolide.

12. A process as claimed in Claim 11 wherein said celery seed is crushed, which celery seed has an average dimension of 1.5 x 1.55 mm and a thickness of 0.5 mm which may be comminuted to provide finely divided particles of an average diameter of 0.1 - 0.2

13. A process as claimed in Claim 12 wherein the comminuted seed is suspended in an organic solvent whereby the ratio of seed to solvent is 1 kg of seed in 5 - 16 litres of solvent.

14. A process as claimed in Claim 13 wherein the organic solvent is ethanol.

15. A process as claimed in Claim 14 wherein the ethanol has a concentration of 95 - 98%.

16. A process as claimed in Claim 14 wherein the ethanol has a concentration of 99.99%.

17. A process as claimed in Claim 13 wherein the comminuted seed is mixed with the solvent under slow agitation until all extractable components in the seed have been fully extracted with the organic solvent.

18. A process as claimed in Claim 17 wherein the particulate celery seed is removed by filtration using filters having a pore size of from 0.2 to 10 microns.

19. A process as claimed in Claim 11 wherein in step (iv) there is a 50% weight loss due to the removal of organic solvent and water to provide a viscous liquid or concentrate.

20. A liquid or concentrate obtainable by the process of claim 11 for use as a therapeutic agent.

21. The use of the therapeutic agent of claim 1 for the manufacture of a medicament for the treatment of inflammatory complaints.

**Patentansprüche**

1. Therapeutisches Mittel zur Behandlung von Entzündungsbeschwerden in Form eines Selleriesamenkonzentrats, welches nach der HPLC-Analyse die in Fig. 2 beschriebenen Peaks A, B und C aufweist, wobei Peak A Sedanolid, Peak B Butylphthalid und Peak C Sedanenolid darstellt.

2. Therapeutisches Mittel nach Anspruch 1, welches im wesentlichen weder Alkohol noch Wasser enthält.

3. Therapeutisches Mittel nach einem vorhergehenden Anspruch, worin eine vierte Komponente in der Zusammensetzung anwesend ist, welche durch die Fläche D in Fig. 2 dargestellt ist.

4. Therapeutisches Mittel nach Anspruch 3, worin das Verhältnis von Sedanolid: Butylphthalid: Sedanenolid und der vierten in der Zusammensetzung anwesenden Komponente etwa 1:15:41:11 beträgt.

5. Therapeutische Formulierung, umfassend ein therapeutisches Mittel nach einem vorhergehenden Anspruch, welche auch einen pharmazeutisch akzeptablen Füllstoff enthält.

6. Therapeutische Formulierung nach Anspruch 5, worin der Füllstoff in der Formulierung auf einer Basis von 5 - 95 Gew.% enthalten ist.

7. Therapeutische Formulierung nach Anspruch 6, worin der Füllstoff in der Formulierung auf einer Basis von 50 Gew.% oder mehr enthalten ist.

8. Therapeutische Formulierung nach Anspruch 7, worin der Füllstoff in der Formulierung auf einer Basis von 75 Gew.% oder mehr enthalten ist.

9. Therapeutische Formulierung nach einem der Ansprüche 5 bis 8, worin der Füllstoff Dicalciumphosphat ist.

10. Therapeutisches Mittel nach Anspruch 2 in Form einer viskosen Flüssigkeit oder eines viskosen Konzentrats.

11. Verfahren zur Herstellung eines Selleriesamenkonzentrats, welches zur Behandlung von Entzündungsbeschwerden nützlich ist, umfassend die Schritte des:

   (i) in Kontaktbringens von Selleriesamen mit einem organischen Lösungsmittel, das lösliche Bestandteile der Selleriesamen zur Erzeugung eines Lösungsmittelextrakts extrahieren kann;
   (ii) Trennens der Mischung aus Selleriesamen und Lösungsmittel zur Entfernung von teilchenförmigem Material;
   (iii) Konzentrierens des Extrakts durch Entfernen des Lösungsmittels zur Entfernung von flüchtigem Material, wobei ein Rückstand von nichtflüchtigen Stoffen verbleibt; und
   (iv) weiteren Konzentrierens des Rückstands unter Vakuum zur Bildung eines Konzentrats, welches nach der HPLC-Analyse die in Fig. 2 beschriebenen Peaks A, B und C aufweist, wobei Peak A Sedanolid, Peak B Butylphthalid und Peak C Sedanenolid darstellt.

12. Verfahren nach Anspruch 11, worin der Selleriesamen grob zerkleinert wird, welcher Selleriesamen eine durchschnittliche Größe von 1,5 x 1,55 mm und eine Dicke von 0,5 mm aufweist, der zur Schaffung von fein verteilten Teilchen mit einem durchschnittlichen Durchmesser von 0,1 bis 0,2 mm feinzerkleinert werden kann.

13. Verfahren nach Anspruch 12, worin der feinzerkleinerte Samen in einem organischen Lösungsmittel suspendiert wird, wobei das Verhältnis von Samen zu Lösungsmittel 1 kg Samen in 5 - 16 Litern Lösungsmittel beträgt.

14. Verfahren nach Anspruch 13, worin das organische Lösungsmittel Ethanol ist.

15. Verfahren nach Anspruch 14, worin das Ethanol eine Konzentration von 95 - 98 % aufweist.

16. Verfahren nach Anspruch 14, worin das Ethanol eine Konzentration von 99,99 % aufweist.

17. Verfahren nach Anspruch 13, worin der feinzerkleinerte Samen unter langsamem Rühren mit dem Lösungsmittel vermischt wird, bis sämtliche extrahierbare Komponenten im Samen mit dem organischen Lösungsmittel vollständig extrahiert sind.

18. Verfahren nach Anspruch 17, worin der teilchenförmige Selleriesamen durch Filtration unter Verwendung von Filtern mit einer Porengröße von 0,2 bis 10 $\mu$m entfernt wird.

19. Verfahren nach Anspruch 11, worin im Schritt (iv) ein 50 %iger Gewichtsverlust aufgrund der Entfernung von organischem Lösungsmittel und Wasser zur Schaffung einer viskosen Flüssigkeit oder eines viskosen Konzentrats stattfindet.

20. Flüssigkeit oder Konzentrat, erhältlich durch das Verfahren nach Anspruch 11, zur Verwendung als therapeutisches Mittel.

21. Verwendung des therapeutischen Mittels nach Anspruch 1 für die Herstellung eines Medikaments zur Behandlung von Entzündungsbeschwerden.

**Revendications**

1. Agent thérapeutique pour le traitement d'une affection inflammatoire sous la forme d'un concentré de graine de céleri qui, à l'analyse HPLC, présente des pointes A, B et C décrites à la figure 2 ci-jointe, dans laquelle la pointe A représente le sedanolide, la pointe B représente le butylphthalide et la pointe C représente le sedanenolide.

2. Agent thérapeutique selon la revendication 1, qui ne contient pratiquement ni alcool ni eau.

3. Agent thérapeutique selon l'une quelconque des revendications précédentes, dans lequel un quatrième composant est présent dans ladite composition qui est représenté par la zone D dans la figure 2 ci-jointe

4. Agent thérapeutique selon la revendication 3, dans lequel le rapport de sedanolide : butylphthalide; sedanenolide et du quatrième composant présent dans ladite composition est approximativement 1:15:41:11.

5. Formulation thérapeutique comprenant un agent thérapeutique comme revendiqué dans l'une quelconque des revendications précédentes, qui comprend également une matière de remplissage pharmaceutiquement acceptable.

6. Formulation thérapeutique selon la revendication 5, dans lequel la matière de remplissage est incluse dans ladite formulation sur une base de 5 - 95% en poids.

7. Formulation thérapeutique selon la revendication 6, dans laquelle ladite matière de remplissage est incluse dans ladite formulation sur la base de 50% ou plus en poids.

8. Formulation thérapeutique selon la revendication 7 dans laquelle ladite matière de remplissage est incluse dans ladite formulation sur une base de 75% ou plus en poids.

9. Formulation thérapeutique selon l'une quelconque des revendications 5 à 8, dans laquelle la matière de remplissage est du diphosphate de chaux.

10. Agent thérapeutique selon la revendication 2 sous la forme d'un liquide visqueux ou concentré.

11. Procédé d'obtention d'un concentré de graines de céleri utile dans le traitement des affections inflammatoires comprenant les étapes consistant à:

   (i) mettre en contact la graine de céleri avec un solvant organique qui peut extraire des composants solubles de la graine de céleri pour produire un extrait de solvant;
   (ii) séparer le mélange de graine de céleri et de solvant pour en retirer un matériau particulaire;
   (iii) concentrer l'extrait en retirant le solvant pour retirer ainsi le matériau volatile laissant un résidu de non-volatiles; et
   (iv) ensuite concentrer ledit résidu sous vide pour fournir un concentré, qui après analyse HPLC, présente des pointes A, B et C décrites dans la figure 2 ci-jointe, dans laquelle la pointe A représente le sedanolide, la pointe B représente le butylphthalide et la pointe C représente le sedanenolide.

12. Procédé selon la revendication 11, dans lequel ladite graine de céleri est écrasée, laquelle graine de céleri possède une dimension moyenne de 1,5 x 1,55 mm et une épaisseur de 0,5 mm, qui peut être réduite en fragments pour procurer finalement des particules divisées d'un diamètre moyen allant de 0,1 à 0,2 mm.

13. Procédé selon la revendication 12, dans lequel la graine réduite en fragments est mise en suspension dans un solvant organique dans lequel le rapport de graine à solvant est de 1 kg de graines dans 5 à 16 litres de solvant.

14. Procédé selon la revendication 13, dans lequel le solvant organique est de l'éthanol.

15. Procédé selon la revendication 14, dans lequel l'éthanol possède une concentration allant de 95 à 98%.

16. Procédé selon la revendication 14, dans lequel l'éthanol possède une concentration de 99,99%.

17. Procédé selon la revendication 13, dans lequel la graine réduite en fragments est mélangée su solvant sous une agitation lente jusqu'à ce que tous les composants extractibles dans la graine aient été extraits complètement avec le solvant organique.

18. Procédé selon la revendication 17, dans lequel la graine de céleri particulaire est retirée par filtration avec utilisation de filtres possédant une dimension de pore allant de 0,2 à 10 microns.

19. Procédé selon la revendication 11, dans lequel il y a, dans la phase (iv), 50% de porte de poids due au retrait du solvant organique et d'eau pour fournir un liquide visqueux ou concentré.

20. Liquide ou concentré pouvant être obtenu par le procédé selon la revendication 11 destiné a être utilisé comme agent thérapeutique,

21. Utilisation d'un agent thérapeutique selon la revendication 1 pour la fabrication d'un médicament pour le traitement des affections inflammatoires.

FIG. 1

FIG. 2

FIG. 3

FIG. 4

FIG. 5

FIG. 6

FIG. 7

FIG. 8

FIG. 9

FIG. 10

FIG. 11

FIG. 12A

FIG. 12B

EP 0 708 651 B1

FIG. 13

EP 0 708 651 B1

EP 0 708 651 B1

FIG. 14 A

FIG. 14B

EP 0 708 651 B1

FIG. 15

FIG. 16A

FIG 16B

FIG. 17

FIG. 18

FIG. 19

RETENTION TIME (min) FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

FIG. 34

FIG. 35

FIG. 36

FIG 37

FIG. 38

FIG. 39

FIG. 40

FIG. 41

FIG. 42